# EUROPÄISCHE PATENTSCHRIFT

(11) **EP 1 812 382 B1**
(45) Veröffentlichungstag und Bekanntmachung des Hinweises auf die Patenterteilung: **23.01.2013**
(21) Anmeldenummer: 05803068.5
(22) Anmeldetag: 27.10.2005
(51) Int. Cl.: C07C 263/10, C07C 265/12, C07C 265/14

(54) **VERFAHREN ZUR HERSTELLUNG VON POLYISOCYANATEN**
METHOD FOR PRODUCING POLYISOCYANATES
PROCEDE POUR PRODUIRE DES POLYISOCYANATES

(30) Priorität: 03.11.2004 DE 102004053662
(43) Veröffentlichungstag der Anmeldung: 01.08.2007
(73) Patentinhaber: BASF SE, 67056 Ludwigshafen (DE)
(72) Erfinder: SESING, Martin, 67165 Waldsee (DE); ROHDE, Thorsten, 68305 Mannheim (DE); STROEFER, Eckhard, 68163 Mannheim (DE); HENKELMANN, Jochem, 68165 Mannheim (DE)
(86) Internationale Anmeldenummer: PCT/EP2005/011490
(87) Internationale Veröffentlichungsnummer: WO 2006/048171

(56) Entgegenhaltungen:
- WO-A-02/02217
- DE-A1- 10 027 779
- US-A1- 2004 024 244

## Beschreibung

Gegenstand der Erfindung ist ein Verfahren zur Herstellung von Polyisocyanaten durch Umsetzung der entsprechenden Amine mit Phosgen.

Polyisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen.

Die kontinuierliche Herstellung der organischen Polyisocyanate durch Reaktion von primären organischen Aminen mit Phosgen ist vielfach beschrieben und wird im großtechnischen Maßstab durchgeführt (s. z.B. Ullmanns Enzyklopädie der Technischen Chemie, and 7 (Polyurethane), 3. neubearbeitete Auflage, Carl Hanser Verlag, München-Wien, S. 76ff (1993)). Insbesondere die aromatischen Isocyanate TDI (Toluylendiisocyanat) und MDI (Methylendiphenyldiisocyanat) bzw. PMDI (Polymethylenpolyphenylenpolyisocyanat) sowie die aliphatischen Isoyanate HDI (Hexamethylendiphenyldiisocyanat) und lsophorondilsocyanat (IPDI) werden großtechnisch hergestellt.

In der Regel ist die kontinuierliche Ausführungsform dieses Verfahrens zweistufig. In der ersten Stufe der Phosgenierung wird das Amin mit Phosgen zu Carbamoylchlorid und Chlorwasserstoff und in einer parallelen Reaktion zu Aminhydrochlorid umgesetzt Die Reaktion zwischen Amin und Phosgen ist sehr schnell, stark exotherm und läuft schon bei sehr niedrigen Temperaturen ab. Um Nebenprodukte- und Feststoffbildung zu minimieren, müssen daher Amin und Phosgen, gegebenenfalls in Mischung mit organischem Lösungsmittel, schnell vermischt werden. Daher erfolgt die erste Phosgenierstufe in der Regel in einem Mischorgan, vorzugsweise einer Düse. Die zweite Stufe der Phosgenierung umfasst sowohl die Zersetzung des üblicherweise als Feststoff vorliegenden Carbamoylchlorids zum gewünschten Isocyanat und Chlorwasserstoff als auch die Phosgenierung des Aminhydrochlorids zum Carbamoylchlorid. Die Temperatur der zweiten Phosgenierstufe ist in der Regel höher als die der ersten. Für die zweite Stufe sind eine Vielzahl von Reaktoren entwickelt worden.

Der bei der Umsetzung gebildete Chlorwasserstoff wird zumeist sehr schnell aus der Reaktionsmischung entfernt, um den Druck im Reaktionssystem zu verringern und das Gleichgewicht der Reaktion in Richtung der Isocyanate zu verschieben.

Die Geschwindigkeit der Umsetzung von Phosgen mit Amin bzw. von Chlorwasserstoff mit Amin hängt vor allem von der Art des herzustellenden Isocyanats und der gewählten Reaktionstemperatur ab.

In der Kalt-Heiß Phosgenierung werden intermediär Aminhydrochloride gebildet, die in langsamer Fest-Flüssig Reaktion abreagieren. Moderne Düsenverfahren, wie beispielsweise in WO 02/02217 und WO 01/91898 beschrieben, arbeiten ausschließlich nach dem Prinzip der Heißphosgenierung, wobei intermediär Aminhydrochlorid-Nanopartikel auftreten, die eine große Oberfläche aufweisen. Diese Partikel reagieren in einer Folgereaktion mit Phosgen ab, wobei das Wertprodukt entsteht. Die Reaktion der Feststoffe mit dem Phosgen verläuft sehr langsam. Außerdem besteht die Gefahr, dass die Feststoffe zu Verstopfungen in der Anlage führen.

Die Herstellung der Isocyanate erfolgt zumeist in Lösung. Dabei werden die Ausgangsstoffe in den Lösungsmitteln gelöst, die gegenüber den Einsatz- und Endprodukten inert sind, die Lösungen miteinander zur Umsetzung gebracht und anschließend das Lösungsmittel abgetrennt.

Als Lösungsmittel werden vorzugsweise chlorierte aromatische Kohlenwasserstoffe wie Dichlorbenzol, Chlorbenzol, Trichlorbenzol oder aromatische oder aliphatische Kohlenwasserstoffe wie Toluol, Xylol, Benzol, Pentan, Hexan, Heptan, Octan, Cyclohexan, Biphenyl, Ketone wie 2-Butanon, Methylisobutylketon, Ester wie Diethylisophtalate, Ethylacetat, Butylacetat, Nitrile wie Acetonitril, oder Sulfolan u.a. verwendet. Hierbei ist jedoch die Löslichkeit der intermediär gebildeten Aminhydrochloride und Carbamoylchloride häufig unzureichend.

Aus DE 11 92 641 und DE 100 27 779 ist bekannt, auch die Isocyanate selbst als Lösungsmittel einzusetzen. Isocyanat als Lösungsmittel hat den Vorteil einer höheren Polarität als die üblicherweise eingesetzten inerten Lösungsmittel. Damit werden intermediär gebildete salzartige Feststoffe besser angelöst. Der Nachteil hierbei ist jedoch die Bildung von Harnstoffen durch die Reaktion der als Lösungsmittel eingesetzten Isocyanate mit den Aminen.

Eine Möglichkeit zur Verbesserung der Löslichkeit der Feststoffe wäre der Einsatz von stark polaren Lösungsmitteln, wie Alkoholen. Dies würde allerdings zu so starken Nebenreaktionen führen, dass es technisch nicht in Frage kommt.

Eine ständige Forderung bei der Herstellung von Isocyanaten durch Umsetzung der entsprechenden Amine mit Phosgen ist die Verringerung der im Reaktionssystem vorhandenen Menge an Phosgen, auch als Phosgen hold-up bezeichnet. Weiterhin ist eine ständige Forderung bei der Herstellung von Polyisocyanaten, die Nebenreaktionen zu verringern und so zu einer höheren Ausbeute und zu Produkten mit einer verbesserten Qualität zu gelangen.

Aufgabe der Erfindung war es, ein Verfahren zur Herstellung von Polyisocyanaten durch Umsetzung von Aminen mit Phosgen zu entwickeln, bei dem die Bildung von Feststoffen minimiert wird, ohne dass es zu Nebenreaktionen kommt. Die Reaktion sollte weiterhin bei niedrigen Drücken und/oder Temperaturen möglich sein. Hieraus resultieren ein geringerer Phosgen hold-up, höhere Raum-Zeit-Ausbeuten und bessere Selektivitäten.

Die Aufgabe konnte überraschenderweise gelöst werden, indem als Lösungsmittel bestimmte ionische Flüssigkeiten eingesetzt werden, die mindestens ein Kation und mindestens ein Anion aufweisen, wobei das Kation der ionischen Flüssigkeit organisch ist.

Gegenstand der Erfindung ist demzufolge ein Verfahren zur Herstellung von Polyisocyanaten durch Umsetzung von primären Aminen mit Phosgen in Anwesenheit eines Lösungsmittels, dadurch gekennzeichnet, dass als Lösungsmittel ionische Flüssigkeiten eingesetzt werden, die mindestens ein Kation und mindestens ein Anion aufweisen, wobei das Kation der ionischen Flüssigkeit organisch ist und ausgewählt ist aus der Gruppe enthaltend quaternäre Ammonium-Kationen, Phosphonium-Kationen, Imidazolium-Kationen, H-Pyrazolium-Kationen, Pyridazinium-lonen, Pyrimidinium-lonen, Pyrazinium-lonen Pyrolidinium-Kationen, Guadinium-Kationen, fünf bis mindestens sechsgliedrige Kationen, die mindestens ein Phosphor- oder Schwefelatom enthalten, und dem 1,8-Diazabicyclo[5.4.0]undec-7-enium-Kation sowie dem 1,8-Diazabicyclo[4.3.0]non-5-enium-Kation sowie Oligo- und Polymere, die diese Kationen enthalten.

Unter ionischen Flüssigkeiten im Sinne der vorliegenden Erfindung werden Verbindungen verstanden, die mindestens ein kationisches Zentrum und mindestens ein anionisches Zentrum aufweisen, insbesondere die mindestens ein Kation und mindestens ein Anion aufweisen, wobei eines der Ionen, insbesondere das Kation, organisch ist.

Ionische Flüssigkeiten sind nach der Definition von Wasserscheid und Keim in: Angewandte Chemie 2000, 112, 3926 - 3945 bei relativ niedrigen Temperaturen schmelzende Salze mit nicht molekularem, ionischem Charakter. Sie sind bereits bei relativ niedrigen Temperaturen flüssig und dabei relativ niedrig viskos. Sie besitzen sehr gute Löslichkeiten für eine große Anzahl organischer, anorganischer und polymerer Substanzen. Darüber hinaus sind sie in der Regel nicht brennbar, nicht korrosiv und haben keinen messbaren Dampfdruck.

Ionische Flüssigkeiten sind Verbindungen, die aus positiven und negativen Ionen gebildet, jedoch insgesamt ladungsneutral sind. Die positiven wie auch die negativen Ionen sind überwiegend einwertig, möglich sind jedoch auch multivalente Anionen und/oder Kationen, beispielsweise mit einer bis fünf, bevorzugt mit einer bis vier, weiter bevorzugt mit einer bis drei und ganz besonders bevorzugt mit einer bis zwei elektrischen Ladungen pro Ion. Die Ladungen können sich an verschiedenen lokalisierten oder delokalisierten Bereichen innerhalb eines Moleküls befinden, also betainartig, oder auch wie ein getrenntes Anion und Kation verteilt sein. Bevorzugt sind solche ionischen Flüssigkeiten, die aus mindestens einem Kation und mindestens einem Anion aufgebaut sind.

Bekannte Einsatzgebiete für ionische Flüssigkeiten sind insbesondere als Lösungsmittel für chemische Reaktionen, als Hilfsmittel zur Abtrennung von Säuren aus chemischen Reaktionsgemischen, wie in DE 10202838 beschrieben, als Hilfsstoffe für die Extraktivrektifikation zur Trennung engsiedender oder azeotroper Gemische, wie in WO 02/074718 beschrieben oder als Wärmeträger in solarthermischen Anlagen, entsprechend der Beschreibung in Proceeding of Solar Forum, 2001, April 21 bis 25, Washington, D.C.

Die erfindungsgemäß eingesetzten ionischen Flüssigkeiten können auch als Gemische verschiedener ionischer Flüssigkeiten verwendet werden.

Bevorzugt sind ionische Flüssigkeiten mit möglichst niedrigem Schmelzpunkt, insbesondere unterhalb von 150°C, weiter bevorzugt unterhalb von 100°C, besonders bevorzugt unterhalb von 80°C.

Die als Reaktionsmedium fungierende ionische Flüssigkeit wird bevorzugt so ausgewählt, dass sie weitgehend inert gegenüber den an der Reaktion teilnehmenden Stoffen ist, bei den Reaktionsbedingungen als Flüssigkeit vorliegt, eine für die Reaktion ausreichende Löslichkeit für die bei der Reaktion entstehenden Produkte und Zwischenprodukte hat, insbesondere eine gute Löslichkeit für das intermediär entstehende Aminhydrochlorid und Carbamoylchlorid aufweist, die korrespondierende Säure des Anions der ionischen Flüssigkeit geringer flüchtig ist als die des bei der Reaktion entstehenden Chlorwasserstoffs, und die Produktabtrennung bevorzugt durch die Ausbildung einer zweiten Phase mit dem Reaktionsprodukt oder durch die Extraktion mit einem weiteren Lösungsmittel, in dem die ionische Flüssigkeit weitgehend unlöslich ist, erfolgen kann.

Bevorzugt werden ionische Flüssigkeiten der allgemeinen Formel

[A]ₙ⁺ [Y]ⁿ⁻

wobei n = 1,2, 3 oder 4 ist und
das Kation [A] ausgewählt ist aus
- quart. Ammonium-Kationen der allgemeinen Formel,

   [NR¹R²R³R]⁺

Phosphonium-Kationen der allgemeinen Formel,

[PR¹R²R³R]⁺

Imidazolium-Kationen der allgemeinen Formel, 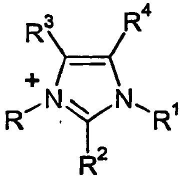 sowie alle, der obigen Formel analogen isomeren Imidazoliniumkationen und Imidazolidiniumkationen,
H-Pyrazolium-Kationen der allgemeinen Formel, 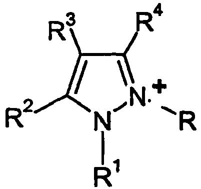 sowie 3H-Pyrazolium-Kationen, 4H-Pyrazolium-Kationen, 1-Pyrazolinium-Kationen, 2-Pyrazolinium-Kationen und 3-Pyrazolinium-Kationen,
- Pyridinium-Kationen der allgemeinen Formel, 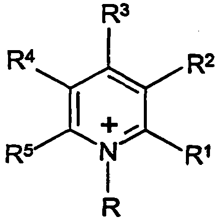
sowie Pyridazinium-, Pyrimidinium- und Pyraziniumionen,

Pyrrolidinium-Kationen der allgemeinen Formel, 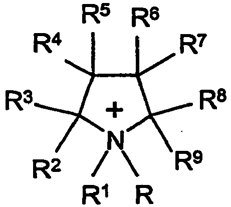 Guanidinium-Kationen der allgemeinen Formel, 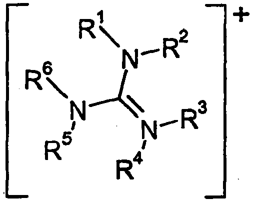
- fünf- bis mindestens sechsgliedrige heterocyclische Kationen, die mindestens ein Phosphor- oder Stickstoffatom sowie gegebenenfalls ein Sauerstoff- oder Schwefelatom aufweisen, wie beispielsweise Thiazolium-, Oxazolium, 1,2,4-Triazolium - oder 1,2,3-Triazolium, besonders bevorzugt solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit ein oder zwei Stickstoffatomen,
- dem 1,8-Diazabicyclo[5.4.0]undec-7-enium-Kation sowie dem 1,8-Diazabicyclo-[4.3.0]non-5-enium-Kation: 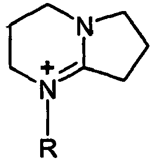
sowie Oligo- und Polymere, die diese Kationen enthalten, wobei die Reste R, R¹, R², R³, R⁴, R⁵, R⁶, R⁷, R⁸ und R⁹ unabhängig voneinander jeweils Wasserstoff, C1 - C18-Alkyl, gegebenenfalls durch ein oder mehrere Sauerstoff- und/oder Schwefelatome und/oder ein oder mehrere substituierte oder unsubstituierte Iminogruppen unterbrochenes C2 - C18-Alkyl, C6 - C12-Aryl, C5 - C12-Cycloalkyl oder einen fünf bis sechsgliedrigen, Sauerstoff , Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

Darin bedeuten gegebenenfalls durch funktionelle Gruppen, Aryl, Alkyl, Aryloxy, Alkyloxy, Halogen, Heteroatome und/oder Heterocyclen substituiertes C₁-C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Di-oxan-2-yl, 2-Methyl-1,3-dioxolan-2-yl, 4-Methyl-1,3-dioxolan-2-yl, 2-lsopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxy-ethyl, 2-Hydroxypropyl, 3-Hydroxypropyl, 4-Hydroxybutyl, 6-Hydroxyhexyl, 2-Amino-ethyl, 2-Aminopropyl, 3-Aminopropyl, 4-Aminobutyl, 6-Aminohexyl, 2-Methylamino-ethyl, 2-Methylaminopropyl, 3-Methylaminopropyl, 4-Methylaminobutyl, 6-Methylamino-hexyl, 2-Dimethylaminoethyl, 2-Dimethylaminopropyl, 3-Dimethylaminopropyl, 4-Di-methylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und,
gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei Kohlenstoffatome.

Substituierte und unsubstituierte Iminogruppen können beispielsweise lmino-, Methylimino-, *iso*-Propylimino, n-Butylimino oder *tert*-Butylimino sein.

Weiterhin bedeuten funktionelle Gruppen Carboxy, Carboxamid, Hydroxy, Di-(C₁-C₄-Alkyl)-amino, C₁-C₄-Alkyloxycarbonyl, Cyano oder C₁-C₄-Alkyloxy,
gegebenenfalls durch funktionelle Gruppen, Aryl, Alkyl, Aryloxy, Alkyloxy, Halogen, Heteroatome und/oder Heterocyclen substituiertes C₆-C₁₂-Aryl beispielsweise Phenyl, Tolyl, Xylyl, α-Naphthyl, α-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch funktionelle Gruppen, Aryl, Alkyl, Aryloxy, Alkyloxy, Halogen, Heteroatome und/oder Heterocyclen substituiertes C₅- C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbomyl oder Norbomenyl,
ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert-Butylthiophenyl und

C₁ bis C₄-Alkyl beispielsweise Methyl, Ethyl, Propyl, Isopropyl, n-Butyl, sec-Butyl oder tert.-Butyl.

Bevorzugt sind R, R¹, R², R³, R⁴, R⁵, R⁶, R⁷, R⁸ und R⁹ unabhängig voneinander Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Benzyl, Acetyl, Dimethylamino, Diethylamino und Chlor.

Zusätzlich Verwendung können gemischte Spezies finden, wie
[A¹]⁺[A²]⁺ [Y]²⁻, [A¹]⁺[A²]⁺[A³]⁺ [Y]³⁻ oder [A¹]⁺[A²]⁺[A³]⁺[A⁴]⁺ [Y]⁴⁻
wobei A¹, A², A³ und A⁴ unabhängig voneinander aus den für [A] genannten Gruppen ausgewählt sind.

Daneben können Verwendung finden gemischte Spezies mit Metallkationen
[A¹]⁺[A²]⁺[A³]⁺[M¹]⁺ [Y]⁴⁻, [A¹]⁺[A²]⁺[M¹]⁺[M²]+ [Y]⁴⁻,
[A¹]⁺[M¹]⁺[M²]⁺M³]⁺ [Y]⁴⁻, [A¹]⁺[A²]⁺[M¹]⁺ [Y]³⁻, [A¹]⁺[M¹]⁺[M²]⁺ [Y]³⁻,
[A¹]⁺[M¹]⁺ [Y]²⁻, [A¹]⁺[A²]⁺[M⁴]²⁺ [Y]⁴⁻, [A¹]⁺[M¹]⁺[M⁴]²⁺ [Y]⁴⁻,
[A¹]⁺[M⁵]³⁺ [Y]⁴⁻, [A¹]⁺[M⁴]²⁺ [Y]³⁻
wobei M¹, M², M³ einwertige Metallkationen, M⁴ zweiwertige Metallkationen und M⁵ dreiwertige Metallkationen darstellen.

Als Anionen sind prinzipiell alle Anionen einsetzbar.

Das Anion [Y] ist bevorzugt ausgewählt aus
der Gruppe der Halogenide bzw. halogenhaltigen Verbindungen der Formel:
Cl⁻, Br⁻, BF₄⁻, PF₆⁻, AlCl₄⁻, Al₂Cl₇⁻, FeCl₄⁻, BCl₄⁻, SbF₆⁻, AsF₆⁻, ZnCl₃⁻, SnCl₃-, CF₃SO₃⁻, (CF₃SO₃)₂N⁻, CF₃CO₂-, CCl_{3C}O₂⁻, CN⁻, SCN⁻, OCN⁻
der Gruppe der Sulfate, Sulfite oder der Sulfonate der allgemeinen Formel:

   SO₄²⁻, HSO₄-, SO₃²⁻, HSO₃⁻, R^{a}OSO₃⁻, R^{a}SO₃⁻
der Gruppe der Phosphate der allgemeinen Formel

   PO₄³⁻, HPO₄²⁻, H₂PO₄-, R^{a}PO₄²⁻, HR^{a}PO₄-, R^{a}R^{b}PO₄⁻
der Gruppe der Phosphonate oder der Phosphinate der allgemeinen Formel:

   RaHPO₃⁻,R^{a}R^{b}PO₂⁻, R^{a}R^{b}PO₃⁻
der Gruppe der Phosphite der allgemeinen Formel:

   PO₃³⁻, HPO₃²⁻, H₂PO₃⁻, R^{a}PO₃²⁻, R^{a}HPO₃⁻_{,} R^{a}R^{b}PO₃⁻
der Gruppe der Phosphonite oder der Phosphinite der allgemeinen Formel:

   R^{a}R^{b}PO₂⁻, R^{a}HPO₂⁻, R^{a}R^{b}PO⁻, R^{a}HPO⁻
der Gruppe der Carbonsäuren der allgemeinen Formel:

   R^{a}COO⁻
der Gruppe der Borate der allgemeinen Formel:

   BO₃³⁻, HBO₃²⁻ , H₂BO₃⁻, R^{a}R^{b}BO₃⁻, R^{a}HBO₃-, R^{a}BO₃²⁻
der Gruppe der Boronate der allgemeinen Formel:

   R^{a}BO₂²⁻, R^{a}R^{b}BO⁻
der Gruppe der Carbonate oder der Kohlensäureester der allgemeinen Formel:

   HCO₃-, CO₃⁻, R^{a}CO₃⁻
der Gruppe der Silikate oder der Kieselsäuresäureester der allgemeinen Formel:
   SiO₄⁴⁻, HSiO₄³⁻, H₂SiO₄²⁻, H₃SiO₄⁻, R^{a}SiO₄³⁻, R^{a}R^{b}SiO₄²⁻ , R^{a}R^{b}R^{c}SiO₄⁻, HR^{a}SiO₄²⁻, H₂R^{a}SiO₄⁻, HR^{a}R^{b}SiO₄⁻
der Gruppe der Alkyl- bzw. Arylsilan-Salze der allgemeinen Formel:
   R^{a}SiO₃³⁻, R^{a}R^{b}SiO₂²⁻, R^{a}R^{b}R^{c}SiO⁻, R^{a}R ^{b}R^{c}SiO₃⁻, RaR^{b}R^{c}SiO₂⁻, R^{a}R^{b}SiO₃²⁻
der Gruppe der Carbonsäureimide, Bis(sulfonyl)imide oder der Sulfonylimide der allgemeinen Formel: 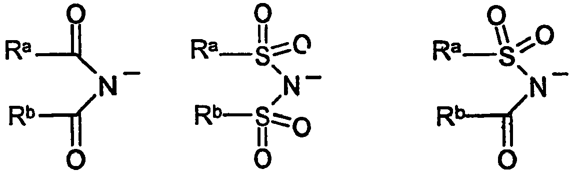
der Gruppe der Alkoxide oder der Aryloxide der allgemeinen Formel:
der Gruppe der komplexen Metallionen wie Fe(CN)₆³⁻, Fe(CN)₆⁴⁻, MnO₄⁻, Fe(CO)₄
und die Reste R^{a}, R^{b}, R^{c} unabhängig voneinander jeweils C1 - C18-Alkyl, gegebenenfalls durch ein oder mehrere Sauerstoff- und/oder Schwefelatome und/oder ein oder mehrere substituierte oder unsubstituierte Iminogruppen unterbrochenes C2 - C18-Alkyl, C6 - C12-Aryl, C5 - C12-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

Darin bedeuten gegebenenfalls durch funktionelle Gruppen, Aryl, Alkyl, Aryloxy, Alkyloxy, Halogen, Heteroatome und/oder Heterocyclen substituiertes C₁- C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethyl-aminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und,
gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4;8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxatetradecyl, 5-Ethoxy-3-oxa-pentyl, 8-Ethoxy-3,6-dioxa-octyl, 11-Ethoxy-3,6,9-trioxaundecyl, 7-Ethoxy-4-oxa-heptyl, 11-Ethoxy-4,8-dioxa-undecyl, 15-Ethoxy-4,8,12-trioxapentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei.

Substituierte und unsubstituierte Iminogruppen können beispielsweise Imino-, Methylimino-, *iso*-Propylimino, n-Butylimino oder *tert*-Butylimino sein.

Weiterhin bedeuten funktionelle Gruppen Carboxy, Carboxamid, Hydroxy, Di-(C₁-C₄-Alkyl)-amino, C₁-C₄-Alkyloxycarbonyl, Cyano oder C₁-C₄-Alkyloxy,
gegebenenfalls durch funktionelle Gruppen, Aryl, Alkyl, Aryloxy, Alkyloxy, Halogen, Heteroatome und/oder Heterocyclen substituiertes C₆ - C₁₂-Aryl beispielsweise Phenyl, Tolyl, Xylyl, α-Naphthyl, α-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch funktionelle Gruppen, Aryl, Alkyl, Aryloxy, Alkyloxy, Halogen, Heteroatome und/oder Heterocyclen substituiertes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butytcyclohexyl, Methoxycyclohexyl, Dimethoxycyclohexyl, Diethoxycyclohexyl, Butylthiocyclohexyl, Chlorcyclohexyl, Dichlorcydohexyl, Dichlorcyclopentyl sowie ein gesättigtes oder ungesättigtes bicyclisches System wie z.B. Norbornyl oder Norbrnenyl,
ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl und

C₁ bis C₄-Alkyl beispielsweise Methyl, Ethyl, Propyl, Isopropyl, n-Butyl, sec-Butyl oder tert.-Butyl.

Bevorzugt sind R¹, R², R³, R⁴ und R⁵ unabhängig voneinander Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino und Chlor.

Die Kationen werden für das erfindungsgemäße Verfahren vorzugsweise ausgewählt aus der Gruppe, enthaltend 1,2,3-Trimethylimidazolium, 1,3,4,5-Tetramethylimidazolium, 1,3,4-Dimethylimidazolium, 1,3,4-Trimethylimidazolium, 1,3-Dibuty!-2-methylimidazolium, 1,3-Dibutylimidazolium, 1,2-Dimethylimidazolium, 1,3-Dimethylimidazolium, 1-Benzyl-3-methylimidazolium, 1-Butyl-2,3-dimethylimidazolium, 1-Butyl-2-ethyl-5-methylimidazolium, 1-Butyl-2-ethylimidazolium, 1-Butyl-2-methylimidazolium, 1-Butyl-3,4,5-trimethylimidazolium, 1-Butyl-3,4-dimethylimidazolium, 1-Butyl-3-ethylimidazolium, 1-Butyl-3-methylimidazolium, 1-Butyl-4-methylimidazolium, 1-Butylimidazolium, 1-Decyl-3-methylimidazolium, 1-Dodecyl-3-methylimidazolium, 1-Ethyl-2,3-dimethylimidazolium, 1-Ethyl-3-methylimidazolium, 1-Hexadecyl-2,3-dimethylimidazolium, 1-Hexadecyl-3-methylimidazolium, 1-Hexyl-2,3-dimethylimidazolium, 1-Hexyl-3-methylimidazolium, 1-Methyl-2-ethylimidazolium, 1-Methyl-3-octylimidazolium, 1-Methylimidazolium, 1-Pentyl-3-methylimidazolium, 1-Phenylpropyl-3-methylimidazolium, 1-Propyl-2,3-dimethylimidazolium, 1-Tetradecyl-3-methylimidazolium, 2,3-Dimethylimidazolium, 2-Ethyl-3,4-dimethylimidazolium, 3,4-Dimethylimidazolium, 1,2-Dimethylpyridinium, 1-Butyl-2-ethyl-6-methylpyridinium, 1-Butyl-2-ethylpyridinium, 1-Butyl-2-methylpyridinium, 1-Butyl-3,4-dimethylpyridinium, 1-Butyl-3,5-dimethylpyridinium, 1-Butyl-3-ethylpyridinium, 1-Butyl-3-methylpyridinium, 1-Butyl-4-methylpyridinium, 1-Butylpyridinium, 1-Ethylpyridinium, 1-Hexyl-3-methylpyridinium, 1-Hexyl-4-methylpyridinium, 1-Hexylpyridinium, 1-Methylpyridinium, 1-Octylpyridinium, 2-Ethyl-1,6-dimethylpyridinium, 2-Ethyl-1-methylpyridinium, 4-Methyl-1-octylpyridinium, 1,1-Dimethylpyrrolidinium, 1-Butyl-1-ethylpyrrolidinium, 1-Butyl-1-methylpyrrolidinium, 1-Ethyl-1-methylpyrrolidinium, 1-Ethyl-3-methylpyrrolidinium, 1-Hexyl-1-methylpyrrolidinium, 1-0ctyl-1-methylpyrrolidinium, Guanidinium, Hexamethylguanidinium, N,N,N',N'-Tetramethyl-N"-ethylguanidinium, N-Pentamethyl-N-isopropylguanidinium, N-Pentamethyl-N-propylguanidinium, Benzyltriphenylphosphonium, Tetrabutylphosphonium, Trihexyl(tetradecyl)phosphonium, Tri-iso-butyl(methyl)phosphonium, Butyltrimethylammonium, Methyltrioctylammonium, Octyltrimethylammonium, Tetrabutylammonium, Tetraethylammonium, Tetramethylammonium, Tributylmethylammonium

Besonders bevorzugte Kationen werden ausgewählt aus der Gruppe, enthaltend 1,2,3-Trimethylimidazolium, 1,2-Dimethylimidazolium, 1-Butyl-2-methylimidazolium, 1-Butyl-4-methylimidazolium, 1,3-Diethylimidazolium, 1-Benzyl-3-methylimidazolium, 1-Butyl-2,3-dimethylimidazolium, 1-Butyl-2-methylimidazolium, 1-Butyl-3-ethylimidazolium, 1-Butyl-3-methylimidazolium, 1-Butylimidazolium, 1-Ethyl-2,3-dimethylimidazolium, 1-Ethyl-3-methylimidazolium, 1-Hexyl-3-methylimidazolium, 1-Methyl-2-ethylimidazolium, 1-Methyl-3-octylimidazolium, 1-Methylimidazolium, 1-Decyl-3-methylimidazolium, 1-Dodecyl-3-methylimidazolium, 1-Butyl-4-methylpyridinium, 1-Butylpyridinium, 1-Ethylpyridinium, 1-Hexylpyridinium, 1-Butyl-1-ethylpyrrolidinium, 1-Butyl-1-methylpyrrolidinium, 1-Ethyl-1-methylpyrrolidinium, 1-Hexyl-1-methylpyrrolidinium, Guanidinium, N,N,N',N'-Tetramethyl-N"-ethylguanidinium, Benzyltriphenylphosphonium, Tetrabutylphosphonium, Butyltrimethylammonium, Methyltrioctylammonium, Tetrabutylammonium, Tributylmethylammonium.

Insbesondere sind die Kationen ausgewählt aus der Gruppe, enthaltend 1,2,3-Trimethylimidazolium, 1,2-Dimethylimidazolium, 1-Butyl-2,3-dimethylimidazolium, 1-Butyl-3-methylimidazolium, 1-Ethyl-2,3-dimethylimidazolium, 1-Ethyl-3-methylimidazolium, 1-Butylimidazolium, 1-Methylimidazolium, 1-Butyl-4-methylpyridinium, 1-Butylpyridinium, Methyltrioctylammonium, Octyltrimethylammonium.

Die Anionen werden für das erfindungsgemäße Verfahren vorzugsweise ausgewählt aus der Gruppe, enthaltend Acetat, Bis(2,4,4-trimethylpentyl)phosphinat, Bis(malonato)borat, Bis(oxalato)borat, Bis(pentafluoroethyl)phosphinat, Bis(phtalato)borat, Bis(salicylato)borat, Bis(trifluoromethansulfonyl)imidat, Bis(trifluoromethansulfonyl)methan, Bis(trifluoromethyl)imidat, Borat, Bromid, Bromoaluminate, Carbonat, Chlorid, Chloroaluminate, Decylbenzolsulfonat, Dichlorocuprat, Dicyanamid, Didecylbenzolsulfonat, Didodecylbenzolsulfonat, Diethylphosphat, Dihydrogenphosphat, Dodecylbenzolsulfonat, Ethylsulfat, Ethylsulfonat, Fluorid, Hexafluorophosphat, Hydrogencarbonat, Hydrogenphosphat, Hydrogensulfat, Hydrogensulfrt, lodid, Methylsulfat, Methylsulfonat, Nitrat, Nitrit, Phosphat, Sulfat, Sulfit, Tetracyanoborat, Tetrafluoroborat, Tetrakis(hydrogensulfato)borat, Tetrakis(methylsulfonato)borat, Thiocyanat, Tosylat, Trichlorozinkat, Trifluoroacetat, Trifluoromethylsulfonat, Tris(heptafluoropropyl)trifluorophosphat, Tris(nonafluorobutyl)trifluorophosphat, Tris(pentafluoroethyl)trifluorophosphat, Tris(pentafluoroethylsulfonyl)trifluorophosphat. Besonders bevorzugt sind die Anionen ausgewählt aus der Gruppe, enthaltend Bis(2,4,4-trimethylpentyl)phosphinat, Bis(malonato)borat, Bis(oxalato)borat, Bis(phtalato)borat, Bis(trifluoromethansulfonyl)imidat, Borat, Chlorid, Chloroaluminate, Decylbenzolsulfonat, Didecylbenzolsulfonat, Didodecylbenzolsulfonat, Dihydrogenphosphat, Dodecylbenzolsulfonat, Ethylsulfat, Ethylsulfonat, Hydrogensulfat, Methylsulfat, Methylsulfonat, Phosphat, Sulfat, Tetrakis(methylsulfonato)borat, Tosylat, Trichlorozinkat.

Insbesondere werden die Anionen ausgewählt aus der Gruppe, enthaltend Chlorid, Chloroaluminate, Ethylsulfat, Methylsulfat, Methylsulfonat, Sulfat, Tosylat.

Bevorzugte ionische Flüssigkeiten für das erfindungsgemäße Verfahren sind ausgewählt aus der Gruppe, enthaltend

1,2,3-Trimethylimidazoliumchlorid, 1,2-Dimethylimidazoliumchlorid, 1-Butyl-2,3-dimethylimidazoliumchlorid, 1-Butyl-3-methylimidazoliumchlorid, 1-Butylimidazoliumchlorid, 1-Methylimidazoliumchlorid ,1-Ethyl-2,3-dimethylimidazoliumchlorid, 1-Ethyl-3-methylimidazoliumchlorid, 1-Butyl-4-methylpyridiniumchlorid, 1-Butylpyridiniumchlorid, Methyltrioctylammoniumchlorid, Octyltrimethylammoniumchlorid, 1,2,3-Trimethylimidazoliumtetrachloroaluminat, 1,2-Dimethylimidazoliumtetrachloroaluminat, 1-Butyl-2,3-dimethylimidazoliumtetrachloroaluminat, 1-Butyl-3-methylimidazolium-tetrachloroaluminat, 1-Ethyl-2,3-dimethylimidazoliumtetrachloroaluminat, 1-Ethyl-3-methylimidazoliumtetrachloroaluminat, 1-Butyl-4-methylpyridiniumtetrachloroaluminat, 1-Butylpyridiniumtetrachloroaluminat, Methyltrioctylammoniumtetrachloroaluminat, Octyltrimethylammoniumtetrachloroaluminat, 1,2,3-Trimethylimidazoliumethylsulfat, 1,2-Dimethylimidazoliumethylsulfat, 1-Butyl-2,3-dimethylimidazoliumethylsulfat, 1-Butyl-3-methylimidazoliumethylsulfat, 1-Ethyl-2,3-dimethylimidazoliumethylsulfat, 1-Ethyl-3-methylimidazoliumethylsulfat, 1-Butyl-4-methylpyridiniumethylsulfat, 1-Butylpyridiniumethylsulfat, Methyltrioctylammoniumethylsulfat, Octyltrimethylammoniumethylsulfat, 1,2,3-Trimethylimidazoliummethylsulfat, 1,2-Dimethylimidazoliummethylsulfat, 1-Butyl-2,3-dimethylimidazoliummethylsulfat, 1-Butyl-3-methylimidazoliummethylsulfat, 1-Ethyl-2,3-dimethylimidazoliummethylsulfat, 1-Ethyl-3-methylimidazoliummethylsulfat, 1-Butyl-4-methylpyridiniummethylsulfat, 1-Butylpyridiniummethylsulfat, Methyltrioctylammoniummethylsulfat, Octyltrimethylammoniummethylsulfat, 1,2,3-Trimethylimidazoliummethylsulfonat, 1,2-Dimethylimidazoliummethylsulfonat, 1-Butyl-2,3-dimethylimidazoliummethylsulfonat, 1-Butyl-3-methylimidazoliummethylsulfonat, 1-Ethyl-2,3-dimethylimidazoliummethylsulfonat, 1-Ethyl-3-methylimidazoliummethylsulfonat, 1-Butyl-4-methylpyridiniummethylsulfonat, 1-Butylpyridiniummethylsulfonat, Methyltrioctylammoniummethylsulfonat, Octyltrimethylammoniummethylsulfonat, 1,2,3-Trimethylimidazoliumsulfat, 1,2-Dimethylimidazoliumsulfat, 1-Butyl-2,3-dimethylimidazoliumsulfat, 1-Butyl-3-methylimidazoliumsulfat, 1-Ethyl-2,3-dimethylimidazoliumsulfat, 1-Ethyl-3-methylimidazoliumsulfat, 1-Butyl-4-methylpyridiniumsulfat, 1-Butylpyridiniumsulfat, Methyltrioctylammoniumsulfat, Octyltrimethylammoniumsulfat, 1,2,3-Trimethylimidazoliumtosylat, 1,2-Dimethylimidazoliumtosylat, 1-Butyl-2,3-dimethylimidazoliumtosylat, 1-Butyl-3-methylimidazoliumtosylat, 1-Ethyl-2,3-dimethylimidazoliumtosylat, 1-Ethyl-3-methylimidazoliumtosylat, 1-Butyl-4-methylpyridiniumtosylat, 1-Butylpyridiniumtosylat, Methyltrioctylammoniumtosylat, Octyltrimethylammoniumtosylat.

Besonders bevorzugte ionischen Flüssigkeiten sind ausgewählt aus der Gruppe, enthaltend 1-Butyl-2,3-dimethylimidazoliumchlorid, 1-Butyl-3-methylimidazoliumchlorid, 1-Butylimidazoliumchlorid , 1-Methylimidazoliumchlorid, 1-Ethyl-2,3-dimethylimidazoliumchlorid, 1-Ethyl-3-methylimidazoliumchlorid, 1-Butyl-4-methylpyridiniumchlorid, 1-Methylpyridiniumchlorid, Methyltrioctylammoniumchlorid, 1-Butyl-2,3-dimethylimidazoliumtetrachloroaluminat, 1-Butyl-3-methylimidazoliumtetrachloroaluminat, 1-Ethyl-2,3-dimethylimidazoliumtetrachloroaluminat, 1-Ethyl-3-methylimidazolium-tetrachloroaluminat, 1-Butyl-4-methylpyridiniumtetrachloroaluminat, 1-Methylpyridiniumtetrachloroaluminat, Methyltrioctylammoniumtetrachloroaluminat, 1-Butyl-2,3-dimethylimidazoliumethylsulfat, 1-Butyl-3-methylimidazoliumethylsulfat, 1-Ethyl-2,3-dimethylimidazoliumethylsulfat, 1-Ethyl-3-methylimidazoliumethylsulfat, 1-Butyl-4-methylpyridiniumethylsulfat, 1-Methylpyridiniumethylsulfat, Methyltrioctylammoniumethylsulfat, 1-Butyl-2,3-dimethylimidazoliummethylsulfat, 1-Butyl-3-methylimidazoliummethylsulfat, 1-Ethyl-2,3-dimethylimidazoliummethylsulfat, 1-Ethyl-3-methylimidazoliummethylsulfat, 1-Butyl-4-methylpyridiniummethylsulfat, 1-Methylpyridiniummethylsulfat, Methyltrioctylammoniummethylsulfat, 1-Butyl-2,3-dimethylimidazoliummethylsulfonat, 1-B utyl-3-methylimidazoliummethylsulfonat, 1-Ethyl-2,3-dimethylimidazoliummethylsulfonat, 1-Ethyl-3-methylimidazoliummethylsulfonat, 1-Butyl-4-methylpyridiniummethylsulfonat, 1-Methylpyridiniummethylsulfonat, Methyltrioctylammoniummethylsulfonat.

Insbesondere sind die ionischen Flüssigkeiten ausgewählt aus der Gruppe, enthaltend 1-Butyl-2,3-dimethylimidazoliumchlorid, 1-Butyl-3-methylimidazoliumchlorid, 1-Butylimidazoliumchlorid , 1-Methylimidazoliumchlorid, 1-Ethyl-3-methylimidazoliumchlorid, 1-Butyl-4-methylpyridiniumchlorid, Methyltrioctylammoniumchlorid, 1-Butyt-2,3-dimethylimidazoliumtetrachloroaluminat, 1-Butyl-3-methylimidazoliumtetrachloroaluminat, 1-Ethyl-3-methylimidazoliumtetrachloroaluminat, 1-Butyl-4-methylpyridiniumtetrachloroaluminat, Methyltrioctylammoniumtetrachloroaluminat, 1-Butyl-2,3-dimethylimidazoliummethylsulfat, 1-Butyl-3-methylimidazoliummethylsulfat, 1-Ethyl-3-methyllmidazoliummethylsulfat, 1-Butyl-4-methylpyridiniummethylsulfat, Methyltrioctylammoniummethylsulfat, 1-Butyl-2,3-dimethylimidazoliummethylsulfonat, 1-Butyl-3-methylimidazoliummethylsulfonat, 1-Ethyl-3-methylimidazoliummethylsulfonat, 1-Butyl-4-methylpyridiniummethylsulfonat, Methyltrioctylammoniummethylsulfonat.

In einer besonderen Ausführungsform der Erfindung wird als Lösungsmittel eine ionische Flüssigkeit in Mischung mit Chlorwasserstoff eingesetzt Das Verhältnis von Chlorwasserstoff zu ionischer Flüssigkeit beträgt in dieser Ausführungsform größer-0 bis 400 Mol-%, bevorzugt 5 bis 300 Mol-%, ganz besonders bevorzugt 10 bis 150 Mol-% bezogen auf die ionische Flüssigkeit.

Überraschenderweise hat sich gezeigt, dass die erfindungsgemäß als Lösungsmittel eingesetzten ionischen Flüssigkeiten ein hohes Lösungsvermögen insbesondere für die Aminhydrochloride und Carbamoylchloride aufweisen; dass sie eine Beschleunigung der Umsetzung, insbesondere der Phosgenierung von Aminhydrochloriden, induzieren; und dass sie durch einfache Destillation oder Extraktion von den Endprodukten abgetrennt werden können.

Nach dem erfindungsgemäßen Verfahren können die üblichen großtechnisch hergestellten Polyisocyanate hergestellt werden. Dies sind beispielsweise die aromatischen Isocyanate TDI (Toluylendiisocyanat) und MDI (Methylendiphenyldiisocyanat), PMDI (Polymethylenpolyphenylenpolyisocyanat) und Mischungen aus MDI und PMDI (Roh-MDI) sowie die aliphatischen Isoyanate HDI (Hexamethylendiphenyldiisocyanat) und Isophorondiisocyanat (IPDI).

Der für das erfindungsgemäße Verfahren vorteilhafte Temperaturbereich hängt unter anderem von der Art und der Menge des Lösemittels und von dem herzustellenden Isocyanat ab. Im allgemeinen liegt in der Mischeinheit eine Temperatur zwischen -20°C und 300°C, bevorzugt zwischen 10°C und 200°C und besonders bevorzugt zwischen 80°C und 150°C vor. Die Temperatur im Reaktor liegt im allgemeinen zwischen 10°C und 360°C und bevorzugt zwischen 40°C und 210°C und besonders bevorzugt bei 80°C und 150°C. Ferner liegt im allgemeinen der Absolutdruck zwischen 0,2 bar und 50 bar, bevorzugt zwischen 0,8 bar und 25 bar, besonders bevorzugt zwischen 1 und 17 bar vor.

Die Verweilzeit der Flüssigkeit in der Mischvorrichtung und im Reaktor liegt in der Summe zwischen 12 s und 20 min, bevorzugt im Bereich von 36 s bis 16 min, und besonders bevorzugt zwischen 60 s und 12 min.

Das molare Verhältnis von eingesetztem Phosgen zu Aminogruppen beträgt 1:1 bis 12:1, bevorzugt 1,1:1 bis 6:1.

Zur Durchführung des erfindungsgemäßen Verfahrens werden die Ausgangsstoffe Amin oder Aminhydrochlorid und Phosgen in der als Lösungsmittel verwendeten ionischen Flüssigkeit gelöst, alternativ hierzu kann auch nur das Amin oder das Aminhydrochlorid in der ionischen Flüssigkeit gelöst werden. Der Strom mit Amin bzw. Aminhydrochlorid gelöst in der ionischen Flüssigkeit wird mit dem Phosgen, welches entweder in der ionischen Flüssigkeit gelöst sein kann oder rein vorliegt vorzugsweise in einer Mischdüse vereinigt. In einer bevorzugten Ausführungsform wird als Mischdüseneinrichtung eine axialsymmetrische Mischrohreinrichtung mit einer axialen Aminzufuhr und einer Phosgenzufuhr, die über zwei nicht axial angeordnete Ringspalte erfolgt, verwendet.

In einer weiteren Ausführungsform der Erfindung kann das Amin auch als Aminhydrochlorid in der ionischen Flüssigkeit gelöst werden und mit dem Phosgen (rein oder in Lösung) vermischt werden. Die Vermischung muss im Gegensatz zu den klassischen Verfahren nicht zwangsläufig sehr schnell durchgeführt werden, da die Weiterreaktion des Isocyanats mit dem Amin zum Harnstoff durch die Zwischenstufe des Hydrochlorides sehr viel langsamer vonstatten geht. Die Reaktion kann hier vorzugsweise bei geringem Druck durchgeführt werden. Als Reaktoren können beispielsweise begaste Rührkessel oder Blasensäulenreaktoren verwendet werden. Der bei der Reaktion entstehende Chlorwasserstoff kann hierbei kontinuierlich aus dem Prozess ausgetragen werden.

In einer weiteren Ausführungsform des erfindungsgemäßen Verfahrens wird zur Herstellung der Phosgen enthaltenden Lösung Phosgen verwendet, das aus der Reaktion zurückgeführt wird und noch Chlorwasserstoff enthält. Bei dieser Ausführungsform kann auf den dem Stand der Technik entsprechenden Verfahrensschritt der Trennung von Phosgen und Chlorwasserstoff nach der Herstellung der Isocyanate verzichtet werden.

Die für das erfindungsgemäße Verfahren eingesetzte Menge der als Lösungsmittel verwendeten ionischen Flüssigkeit beträgt im allgemeinen 10 bis 1000 Gew.-%, bevorzugt 50 bis 500 Gew.-%, mehr bevorzugt 100 bis 400 Gew.-%, bezogen auf die eingesetzte Menge an Amin.

Nach der Reaktion wird das Stoffgemisch, bevorzugt mittels einfacher Phasentrennung, gegebenenfalls durch Zugabe eines weiteren Lösungsmittels, in Isocyanat, Lösungsmittel und Gasphase (aus Phosgen und Chlorwasserstoff) aufgetrennt. Geringe Mengen von Isocyanat, die in der ionischen Flüssigkeit verbleiben, können, wenn notwendig, mittels zusätzlicher Extraktion oder auch Kristallisation von der ionischen Flüssigkeit getrennt werden. Das Isocyanat kann von Nebenprodukten durch die gängigen Reinigungsverfahren befreit werden. Phosgen und Chlorwasserstoff können beispielsweise destillativ voneinander getrennt werden.

Die abgetrennte ionische Flüssigkeit kann anschließend wieder als Lösungsmittel zurückgeführt werden.

Durch die erfindungsgemäße Verwendung ionischer Flüssigkeiten lässt sich die Bildung von Feststoffen verhindern und so der apparative Aufwand zur Beherrschung von Feststoffen einsparen. Zudem reagieren die gebildeten Aminhydrochloride schneller mit dem Phosgen. Des weiteren ist die Reaktionsführung bei niedrigen Drücken und / oder Temperaturen möglich. Hieraus resultieren ein geringerer Phosgen hold-up, höhere RZA und bessere Selektivitäten.

Die Erfindung soll an den nachfolgenden Beispielen näher beschrieben werden.

### Beispiel 1: Herstellung von MDI in Monochlorbenzol (MCB)

In einem 500 ml Vierhalskolben mit Rührer, Innenthermometer, getauchtem Einleitungsrohr für Phosgen und beheizter Zulaufleitung wurden 75 g MCB vorgelegt. Es wurde bei 120°C mit Phosgen gesättigt. Binnen 1 h wurde unter weiterem Einleiten von Phosgen eine Lösung von MDA in MCB (50 g MDA in 90 g MCB) zudosiert. Nach erfolgter Umsetzung wurde mit Stickstoff phosgenfrei gestrippt.

Im feststoffhaltigen Rohaustrag (ca. 60 Gew.-%, laut Elementaranalyse Aminhydrochlorid) war ein Umsatz von 44 % zu erkennen.

### Beispiel 2: Herstellung von MDI in Methylimidazoliumchlorid

In einem 500 ml Vierhalskolben mit Rührer, Innenthermometer, getauchtem Einleitungsrohr für Phosgen und beheizter Zulaufleitung wurden 75 g Methylimidazoliumchlorid (MIA-HCl) vorgelegt. Es wurde bei 130°C mit Phosgen gesättigt. Binnen 1 h wurde unter weiterem Einleiten von Phosgen eine Lösung von MDA in MIA-HCl (50 g MDA in 88 g MIA-HCl) zudosiert. Nach erfolgter Umsetzung wurde mit Stickstoff phosgenfrei gestrippt.

Im Gaschromatogramm des homogenen Rohaustrages war vollständiger Umsatz zu erkennen.

### Beispiel 3: Herstellung von MDI in 1-Butyl-3-methylimidazoliumchlorid

In einem 500 ml Vierhalskolben mit Rührer, Innenthermometer, getauchtem Einleitungsrohr für Phosgen und beheizter Zulaufleitung wurden 200 g 1-Butyl-3-methylimidazoliumchlorid (BMIM-CI) vorgelegt. Es wurde bei 120°C mit Phosgen gesättigt Binnen 4 h wurde unter weiterem Einleiten von Phosgen eine Lösung von MDA in BMIM-CI (50 g MDA in 100 g BMIM-CI) zudosiert. Nach erfolgter Umsetzung wurde mit Stickstoff phosgenfrei gestrippt.

Im Gaschromatogramm des homogenen Rohaustrages war vollständiger Umsatz zu erkennen.

### Beispiel 4: Herstellung von HDI in 1-Butyl-3-methylimidazoliumchlorid

In einem 500 ml Vierhalskolben mit Rührer, Innenthermometer, getauchtem Einleitungsrohr für Phosgen und beheizter Zulaufleitung wurden 200 g Butyl-methylimidazolium-chlorid (BMIM-CI) vorgelegt. Es wurde bei 120°C mit Phosgen gesättigt Binnen 3 h wurde unter weiterem Einleiten von Phosgen eine Lösung von HDA in BMIM-CI (65 g HDA in 100 g BMIM-CI) zudosiert. Nach erfolgter Umsetzung wurde mit Stickstoff phosgenfrei gestrippt.

Im Gaschromatogramm des homogenen Rohaustrages war vollständiger Umsatz zu erkennen.

### Beispiel 5: Herstellung von MDI in Methylimidazoliumchlorid x HCl

In einem 500 ml Vierhalskolben mit Rührer, Innenthermometer, getauchtem Einleitungsrohr für Phosgen und beheizter Zulaufleitung wurden 75 g Methylimidazoliumchlorid (MIA-HCI) vorgelegt. Es wurde bei 120°C mit Chlorwasserstoff-Gas gesättigt. Anschließend wurde bei 120°C mit Phosgen gesättigt. Binnen 1 h wurde unter weiterem Einleiten von Phosgen eine Lösung von MDA in MIA-HCI, welches bei Raumtemperatur mit HCI-Gas gesättigt worden war, (50 g MDA in 88 g MIA x 1.8 HCI) zudosiert. Nach erfolgter Umsetzung wurde mit Stickstoff phosgenfrei gestrippt.

Im Gaschromatogramm des homogenen Rohaustrages war vollständiger Umsatz zu erkennen.

### Beispiel 6: Herstellung von MDI in 1-Ethyl-3-methylimidazoliumchlorid x HCI

In einem 500 ml Vierhalskolben mit Rührer, Innenthermometer, getauchtem Einleitungsrohr für Phosgen und beheizter Zulaufleitung wurden 75 g 1-Ethyl-3-methylimidazoliumchlorid (EMIM-CI) vorgelegt. Es wurde bei 120°C mit Chlorwasserstoff-Gas gesättigt. Anschließend wurde bei 120°C mit Phosgen gesättigt Binnen 1 h wurde unter weiterem Einleiten von Phosgen eine Lösung von MDA in EMIM-CI, welches bei Raumtemperatur mit HCI-Gas gesättigt worden war, (50 g MDA in 93 g EMIM-Cl x 1.3 HCI) zudosiert. Nach erfolgter Umsetzung wurde mit Stickstoff phosgenfrei gestrippt. Im Gaschromatogramm des homogenen Rohaustrages war vollständiger Umsatz zu erkennen.

### Beispiel 7: Herstellung von MDI in Ethylmethylimidazoliumchlorid

In einem 400 ml Druckautoklaven wurden 2,0 g MDAxHCl in 100,5 g EMIM CI (Ethylmethylimidazoliumchlorid) vorgelegt. Zu dieser Lösung werden 7,2 g Phosgen bei 120°C zudosiert. Die Phosgenierung findet unter dem Eigendruck des Reaktionssystems bei der Reaktionstemperatur statt.

| Zeit s | Beispiel 7 Ausbeute % | Beispiel 8 Ausbeute % |
|---|---|---|
| 39 | 34 | 28 |
| 58 | 57 | 34 |
| 86 | 84 | 38 |

### Beispiel 8: Herstellung von MDI in Monochlorbenzol

In einem 400 ml Druckautoklaven wurden 2,0 g MDAxHCl in 100 g Monochlorbenzol vorgelegt. Zu dieser Lösung werden 7,5 g Phosgen bei 120°C zudosiert. Die Phosgenierung findet unter dem Eigendruck des Reaktionssystems bei der Reaktionstemperatur statt.

## Patentansprüche

1. Verfahren zur Herstellung von Polyisocyanaten durch Umsetzung von primären Aminen mit Phosgen in Anwesenheit eines Lösungsmittels, **dadurch gekennzeichnet, dass** als Lösungsmittel ionische Flüssigkeiten die mindestens ein Kation und mindestens ein Anion aufweisen, und das Kation der ionischen Flüssigkeit organisch ist, eingesetzt werden, wobei das Kation der ionischen Flüssigkeit ausgewählt ist aus der Gruppe enthaltend quaternäre Ammonium-Kationen, Phosphonium-Kationen, Imidazolium-Kationen, H-Pyrazolium-Kationen, Pyridazinium-lonen, Pyrimidinium-lonen, Pyrazinium-lonen Pyrolidinium-Kationen, Guadinium-Kationen, fünf- bis mindestens sechsgliedrige Kationen, die mindestens ein Phosphor- oder Schwefelatom enthalten, und dem 1,8-Diazabicyclo[5.4.0]undec-7-enium-Kation sowie dem 1,8-Diazabicyclo[4.3.0]non-5-enium-Kation sowie Oligo- und Polymere, die diese Kationen enthalten.

2. Verfahren nach Anspruch 1, **dadurch gekennzeichnet, dass** das Kation ausgewählt ist aus der Gruppe, enthaltend 1,2,3-Trimethylimidazolium, 1,3,4,5-Tetramethylimidazolium, 1,3,4-Dimethylimidazolium, 1,3,4-Trimethylimidazolium, 1,3-Dibutyl-2-methylimidazolium, 1,3-Dibutylimidazolium, 1,2-Dimethylimidazolium, 1,3-Dimethylimidazolium, 1-Benzyl-3-methylimidazolium, 1-Butyl-2,3-dimethylimidazolium, 1-Butyl-2-ethyl-5-methylimidazolium, 1-Butyl-2-ethylimidazolium, 1-Butyl-2-methylimidazolium, 1-Butyl-3,4,5-trimathylimidazolium, 1-Butyl-3,4-dimethylimidazolium, 1-Butyl-3-ethylimidazolium, 1-Butyl-3-methylimidazolium, 1-Butyl-4-methylimidazolium, 1-Butylimidazolium, 1-Decyl-3-methylimidazolium, 1-Dodecyl-3-methylimidazolium, 1-Ethyl-2,3-dimethylimidazolium, 1-Ethyl-3-methylimidazolium, 1-Hexadecyl-2,3-dimethylimidazolium, 1-Hexadecyl-3-methylimidazolium, 1-Hexyl-2,3-dimethylimidazolium, 1-Hexyl-3-methylimidazolium, 1-Methyl-2-ethylimidazolium, 1-Methyl-3-octylimidazolium, 1-Methylimidazolium, 1-Pentyl-3-methylimidazolium, 1-Phenylpropyl-3-methylimidazolium, 1-Propyl-2,3-dimethylimidazolium, 1-Tetradecyl-3-methylimidazolium, 2,3-Dimethylimidazolium, 2-Ethyl-3,4-dimethylimidazolium, 3,4-Dimethylimidazolium, 1,2-Dimethylpyridinium, 1-Butyl-2-ethyl-6-methylpyridinium, 1-Butyl-2-ethylpyridinium, 1-Butyl-2-methylpyridinium, 1-Butyl-3,4-dimethylpyridinium, 1-Butyl-3,5-dimethylpyridinium, 1-Butyl-3-ethylpyridinium, 1-Butyl-3-methylpyridinium, 1-Butyl-4-methylpyridinium, 1-Butylpyridinium, 1-Ethylpyridinium, 1-Hexyl-3-methylpyridinium, 1-Hexyl-4-methylpyridinium, 1-Hexylpytidinium, 1-Methylpyridinium, 1-Octylpyridinium, 2-Ethyl-1,6-dimethylpyridinium, 2-Ethyl-1-methylpyridinium, 4-Methyl-1-octylpyridinium, 1,1-Dimethylpyrrolidinium, 1-Butyl-1-ethylpyrrolidinium, 1-Butyl-1-methylpyrrolidinium, 1-Ethyl-1-methylpyrrolidinium, 1-Ethyl-3-methylpyrrolidinium, 1-Hexyl-1-methylpyrrolidinium, 1-Octyl-1-methytpyrrolidinium, Guanidinium, Hexamethylguanidinium, N,N,N',N'-Tetramethyl-N"-ethylguanidinium, N-Pentamethyl-N-isopropylguanidinium, N-Pentamethyl-N-propylguanidinium, Benzyltriphenylphosphonium, Tetrabutylphosphonium, Trihexyl(tetradecyl)-phosphonium, Tri-iso-butyl(methyl)phosphonium, Butyltrimethylammonium, Methyltrioctylammonium, Octyltrimethylammonium, Tetrabutylammonium, Tetraethylammonium, Tetramethylammonium, Tributylmethylammonium.

3. Verfahren nach Anspruch 1, **dadurch gekennzeichnet, dass** das Kation ausgewählt ist aus der Gruppe, enthaltend 1,2,3-Trimethylimidazolium, 1,2-Dimethylimidazolium, 1-Butyl-2-methylimidazolium, 1-Butyl-4-methylimidazolium, 1,3-Diethylimidazolium, 1-Benzyl-3-methylimidazolium, 1-Butyl-2,3-dimethylimidazolium, 1-Butyl-2-methylimidazolium, 1-Butyl-3-ethylimidazolium, 1-Butyl-3-methylimidazolium, 1-Butylimidazolium, 1-Ethyl-2,3-dimethylimidazolium, 1-Ethyl-3-methylimidazolium, 1-Hexyl-3-methylimidazolium, 1-Methyl-2-ethylimidazolium, 1-Methyl-3-octylimidazolium, 1-Methylimidazolium, 1-Decyl-3-methylimidazolium, 1-Dodecyl-3-methylimidazolium, 1-Butyl-4-methylpyridinium, 1-Butylpyridinium, 1-Ethylpyridinium, 1-Hexylpyridinium, 1-Butyl-1-ethylpyrrolidinium, 1-Butyl-1-methylpyrrolidinium, 1-Ethyl-1-methylpyrrolidinium, 1-Hexyl-1-methylpyrrolidinium, Guanidinium, N,N,N',N'-Tetramethyl-N"-ethylguanidinium, Benzyltriphenylphosphonium, Tetrabutylphosphonium, Butyltrimethylammonium, Methyltrioctylammonium, Tetrabutylammonium, Tributylmethylammonium.

4. Verfahren nach Anspruch 1, **dadurch gekennzeichnet, dass** das Kation ausgewählt ist aus der Gruppe, enthaltend 1,2,3-Trimethylimidazolium, 1,2-Dimethylimidazolium, 1-Butyl-2,3-dimethylimidazolium, 1-Butyl-3-methylimidazolium, 1-Butylimidazolium, 1-Methylimidazolium, 1-Ethyl-2,3-dimethylimidazolium, 1-Ethyl-3-methylimidazolium, 1-Butyl-4-methylpyridinium, 1-Butylpyridinium, Methyltrioctylammonium, Octyltrimethylammonium.

5. Verfahren nach Anspruch 1, **dadurch gekennzeichnet, dass** das Anion ausgewählt ist aus der Gruppe, enthaltend Halogenide bzw. halogenhaltigen Verbindungen, Sulfate, Sulfite und Sulfonate, Phosphate, Phosphite, Phosphonite und Phosphinite, Carbonsäuren, Borate, Boronate, Carbonate und Kohlensäureester, Silikate und Kieselsäuresäureester, Alkyl- bzw. Arylsilan-Salze, Carbonsäureimide, Bis(sulfonyl)imide und Sulfonylimide, Alkoxide und Aryloxide sowie komplexe Metallionen.

6. Verfahren nach Anspruch 1, **dadurch gekennzeichnet, dass** das Anion ausgewählt ist aus der Gruppe, enthaltend Acetat, Bis(2,4,4-trimethylpentyl)phosphinat, Bis(malonato)borat, Bis(oxalato)borat, Bis(pentafluoroethyl)phosphinat, Bis(phtalato)borat, Bis(salicylato)borat, Bis(trifluoromethansulfanyl)imidat, Bis(trifluoromethansulfonyl)methan, Bis(trifluoromethyl)imidat, Borat, Bromid, Bromoaluminate, Carbonat, Chlorid, Chloroaluminate, Decylbenzolsulfonat, Dichlorocuprat, Dicyanamid, Didecylbenzolsulfonat, Didodecylbenzolsulfonat, Diethylphosphat, Dihydrogenphosphat, Dodecylbenzolsulfonat, Ethylsulfat, Ethylsulfonat, Fluorid, Hexafluorophosphat, Hydrogencarbonat, Hydrogenphosphat, Hydrogensulfat, Hydrogensulfit, Iodid, Methylsulfat, Methylsulfonat, Nitrat, Nitrit, Phosphat, Sulfat, Sulfit, Tetracyanoborat, Tetrafluoroborat, Tetrakis(hydrogensulfato)borat, Tetrakis(methylsulfonato)borat, Thiocyanat, Tosylat, Trichlorozinkat, Trifluoroacetat, Trifluoromethylsulfonat, Tris(heptafluoropropyl)trifluorophosphat, Tris(nonafluorobutyl)trifluorophosphat, Tris(pentafluoroethyl)trifluorophosphat, Tris(pentafluoroethylsulfonyl)trifluorophosphat.

7. Verfahren nach Anspruch 1, **dadurch gekennzeichnet, dass** das Anion ausgewählt ist aus der Gruppe, enthaltend Bis(2,4,4-trimethylpentyl)phosphinat, Bis(malonato)borat, Bis(oxalato)borat, Bis(phtalato)borat, Bis(trifluoromethansulfonyl)imidat, Borat, Chlorid, Chloroaluminate, Decylbenzolsulfonat, Didecylbenzolsulfonat, Didodecylbenzolsulfonat, Dihydrogenphosphat, Dodecylbenzolsulfonat, Ethylsulfat, Ethylsulfonat, Hydrogensulfat, Methylsulfat, Methylsulfonat, Phosphat, Sulfat, Tetrakis(methylsulfonato)borat, Tosylat, Trichlorozinkat.

8. Verfahren nach Anspruch 1, **dadurch gekennzeichnet, dass** das Anion ausgewählt ist aus der Gruppe, enthaltend Chlorid, Chloroaluminate, Ethylsulfat, Methylsulfat, Methylsulfonat, Sulfat, Tosylat.

9. Verfahren nach Anspruch 1, **dadurch gekennzeichnet, dass** die ionische Flüssigkeit ausgewählt ist aus der Gruppe, enthaltend 1,2,3-Trimethylimidazoliumchlorid, 1,2-Dimethylimidazoliumchlorid, 1-Butyl-2,3-dimethylimidazoliumchlorid, 1-Butyl-3-methylimidazoliumchlorid, 1-Butylimidazoliumchlorid, 1-Methylimidazoliumchlorid, 1-Ethyl-2,3-dimethylimidazoliumchlorid, 1-Ethyl-3-methylimidazoliumchlorid, 1-Butyl-4-methylpyridiniumchlorid, 1-Butylpyridiniumchlorid, Methyltrioctylammoniumchlorid, Octyltrimethylammoniumchlorid, 1,2,3-Trimethylimidazoliumtetrachloroaluminat, 1,2-Dimethylimidazoliumtetrachloroaluminat, 1-Butyl-2,3-dimethylimidazoliumtetrachloroaluminat, 1-Butyl-3-methylimidazoliumtetrachloroaluminat, 1-Ethyl-2,3-dimethylimidazoliumtetrachloroaluminat, 1-Ethyl-3-methylimidazoliumtetrachloroaluminat, 1-Butyl-4-methylpyridiniumtetrachloroaluminat, 1-Butylpyridiniumtetrachloroaluminat, Methyltrioctylammoniumtetrachloroaluminat, Octyltrimethylammoniumtetrachloroaluminat, 1,2,3-Trimethylimidazoliumethylsulfat, 1,2-Dimethylimidazoliumethylsulfat, 1-Butyl-2,3-dimethylimidazoliumethylsulfat, 1-Butyl-3-methylimidazoliumethylsulfat, 1-Ethyl-2,3-dimethylimidazoliumethylsulfat, 1-Ethyl-3-methylimidazoliumethylsulfat, 1-Butyl-4-methylpyridiniumethylsulfat, 1-Butylpyridiniumethylsulfat, Methyltrioctylammoniumethylsulfat, Octyltrimethylammoniumethylsulfat, 1,2,3-Trimethylimidaaoliummethylsulfat, 1,2-Dimethylimidazoliummethylsulfat, 1-Butyl-2,3-dimethylimidazoliummethylsulfat, 1-Butyl-3-methyl-imidazoliummethylsulfat, 1-Ethyl-2,3-dimethylimidazoliummethylsulfat, 1-Ethyl-3-methylimidazoliummethylsulfat, 1-Butyl-4-methylpyridiniummethylsulfat, 1-Butylpyridiniummethylsulfat, Methyltrioctylammoniummethylsulfat, Octyltrimethylammoniummethylsulfat, 1,2,3-Trimethylimidazaliummethylsulfonat, 1,2-Dimethylimidazoliummethylsulfonat, 1-Butyl-2,3-dimethylimidazoliummethylsulfonat, 1-Butyl-3-methylimidazoliummethylsulfonat, 1-Ethyl-2,3-dimethylimidazoliummethylsulfonat, 1-Ethyl-3-methylimidazoliummethylsulfonat, 1-Butyl-4-methylpyridiniummethylsulfonat, 1-Butylpyridiniummethylsulfonat, Methyltrioctylammoniummethylsulfonat, Octyltrimethylammoniummethylsulfonat, 1,2,3-Trimethylimidazoliumsulfat, 1,2-Dimethylimidazoliumsulfat, 1-Butyl-2,3-dimethylimidazoliumsulfat, 1-Butyl-3-methylimidazoliumsulfat, 1-Ethyl-2,3-dimethylimidazoliumsulfat, 1-Ethyl-3-methylimidazoliumsulfat, 1-Butyl-4-methylpyridiniumsulfat, 1-Butylpyridiniumsulfat, Methyltrioctylammoniumsulfat, Octyltrimethylammoniumsulfat, 1,2,3-Trimethylimidazoliumtosylat, 1,2-Dimethylimidazoliumtosylat, 1-Butyl-2,3-dimethylimidazoliumtosylat, 1-Butyl-3-methylimidazoliumtosylat, 1-Ethyl-2,3-dimethylimidazoliumtosylat, 1-Ethyl-3-methylimidazoliumtosylat, 1-Butyl-4-methylpyridiniumtosylat, 1-Butylpyridiniumtosylat, Methyltrioctylammoniumtosylat, Octyltrimethylammoniumtosylat.

10. Verfahren nach Anspruch 1, **dadurch gekennzeichnet, dass** die ionische Flüssigkeit ausgewählt ist aus der Gruppe, enthaltend 1-Butyl-2,3-dimethylimidazoliumchlorid, 1-Butyl-3-methylimidazoliumchlorid, 1-Butylimidazoliumchlorid, 1-Methylimidazoliumchlorid, 1-Ethyl-2,3-dimethylimidazoliumchlorid, 1-Ethyl-3-methylimidazoliumchlorid, 1-Butyl-4-methylpyridiniumchlorid, 1-Methylpyridiniumchlorid, Methyltrioctylammoniumchlorid, 1-Butyl-2,3-dimethylimidazoliumtetrachloroaluminat, 1-Butyl-3-methylimidazoliumtetrachloroaluminat, 1-Ethyl-2,3-dimethylimidazoliumtetrachloroaluminat, 1-Ethyl-3-methylimidazoliumtetrachloroaluminat, 1-Butyl-4-methylpyridiniumtetrachloroaluminat, 1-Methylpyridiniumtetrachloroaluminat, Methyltrioctylammoniumtetrachloroaluminat, 1-Butyl-2,3-dimethylimidazoliumethylsulfat, 1-Butyl-3-methylimidazoliumethylsulfat, 1-Ethyl-2,3-dimethylimidazoliumethylsulfat, 1-Ethyl-3-methylimidazoliumethylsulfat, 1-Butyl-4-methylpyridiniumethylsulfat, 1-Methylpyridiniumethylsulfat, Methyltrioctylammoniumethylsulfat, 1-Butyl-2,3-dimethylimidazoliummethylsulfat, 1-Butyl-3-methylimidazoliummethylsulfat, 1-Ethyl-2,3-dimethylimidazoliummethylsulfat, 1-Ethyl-3-methylimidazoliummethylsulfat, 1-Butyl-4-methylpyridiniummethylsulfat, 1-Methylpyridiniummethylsulfat, Methyltrioctylammoniummethylsulfat, 1-Butyl-2,3-dimethylimidazoliummethylsulfonat, 1-Butyl-3-methylimidazoliummethylsulfonat, 1-Ethyl-2,3-dimethylimidazoliummethylsulfonat, 1-Ethyl-3-methylimidazoliummethylsulfonat, 1-Butyl-4-methylpyridiniummethylsulfonat, 1-Methylpyridiniummethylsulfonat, Methyltrioctylammoniummethylsulfonat.

11. Verfahren nach Anspruch 1, **dadurch gekennzeichnet, dass** die ionischen Flüssigkeit ausgewählt ist aus der Gruppe, enthaltend 1-Butyl-2,3-dimethylimidazoliumchlorid, 1-Butyl-3-methylimidazoliumchlorid, 1-Butylimidazoliumchlorid, 1-Methylimidazoliumchlorid, 1-Ethyl-3-methylimidazoliumchlorid, 1-Butyl-4-methylpyridiniumchlorid, Methyltrioctylammoniumchlorid, 1-Butyl-2,3-dimethylimidazoliumtetrachloroaluminat, 1-Butyl-3-methylimidazoliumtetrachloroaluminat, 1-Ethyl-3-methylimidazoliumtetrachloroaluminat, 1-Butyl-4-methylpyridiniumtetrachloroaluminat, Methyltrioctylammoniumtetrachloroaluminat, 1-Butyl-2,3-dimethylimidazoliummethylsulfat, 1-Butyl-3-methylimidazolium-methylsulfat, 1-Ethyl-3-methylimidazoliummethylsulfat, 1-Butyl-4-methylpyridiniummethylsulfat, Methyltrioctylammoniummethylsulfat, 1-Butyl-2,3-dimethylimidazoliummethylsulfonat, 1-Butyl-3-methylimidazoliummethylsulfonat, 1-Ethyl-3-methylimidazoliummethylsulfonat, 1-Butyl-4-methylpyridiniummethylsulfonat, Methyltrioctylammoniummethylsulfonat.

12. Verfahren nach Anspruch 1, **dadurch gekennzeichnet, dass** die ionische Flüssigkeit ausgewählt ist aus der Gruppe, enthaltend 1-Butyl-3-methylimidazoliumchlorid, 1-Ethyl-3-methylimidazoliumchlorid, 1-Butyl-4-methylpyridiniumchlorid, 1-Butylimidazoliumchlorid, 1-Methylimidazoliumchlorid.

13. Verfahren nach Anspruch 1, **dadurch gekennzeichnet, dass** das Amin als Aminhydrochlorid in der ionischen Flüssigkeit gelöst vorliegt.

14. Verfahren nach Anspruch 1, **dadurch gekennzeichnet, dass** die ionische Flüssigkeit in Mischung mit Chlorwasserstoff eingesetzt wird.

15. Verfahren nach Anspruch 14, **dadurch gekennzeichnet, dass** das Verhältnis von Chlorwasserstoff zu ionischer Flüssigkeit größer 0 bis 400 Mol-% beträgt.

16. Verfahren nach Anspruch 14, **dadurch gekennzeichnet, dass** das Verhältnis von Chlorwasserstoff zu ionischer Flüssigkeit 5 bis 300 Mol-% beträgt.

17. Verfahren nach Anspruch 14, **dadurch gekennzeichnet, dass** das Verhältnis von Chlorwasserstoff zu ionischer Flüssigkeit 10 bis 150 Mol-% beträgt.

## Claims

1. A process for preparing polyisocyanates by reacting primary amines with phosgene in the presence of a solvent, wherein ionic liquids which have at least one cation and at least one anion, and the cation of the ionic liquid is organic, are used as solvents,
where the cation of the ionic liquid is selected from the group consisting of quaternary ammonium cations, phosphonium cations, imidazolium cations, H-pyrazolium cations, pyridazinium ions, pyrimidinium ions, pyrazinium ions, pyrrolidinium cations, guadinium cations, five- to at least six-membered cations containing at least one phosphorus or sulfur atom and the 1,8-diazabicyclo[5.4.0]undec-7-enium cation and the 1,8-diazabicyclo[4.3.0]non-5-enium cation and oligomers and polymers in which these cations are present.

2. The process according to claim 1, wherein the cation is selected from the group consisting of 1,2,3-trimethylimidazolium, 1,3,4,5-tetramethylimidazolium, 1,3,4-dimethylimidazolium, 1,3,4-trimethylimidazolium, 1,3-dibutyl-2-methylimidazolium, 1,3-dbutylimidazolium, 1,2-dimethylimidazolium, 1,3-dimethylimidazolium, 1-benzyl-3-methylimidazolium, 1-butyl-2,3-dimethylimidazolium, 1-butyl-2-ethyl-5-methylimidazolium, 1-butyl-2-ethylimidazolium, 1-butyl-2-methylimidazolium, 1-butyl-3,4,5-trimethylimidazolium, 1-butyl-3,4-dimethylimidazolium, 1-butyl-3-ethylimidazolium, 1-butyl-3-methylimidazolium, 1-butyl-4-methylimidazolium, 1-butylimidazolium, 1-decyl-3-methylimidazolium, 1-dodecyl-3-methylimidazolium, 1-ethyl-2,3-dimethylimidazolium, 1-ethyl-3-methylimidazolium, 1-hexadecyl-2,3-dimethylimidazolium, 1-hexadecyl-3-methylimidazolium, 1-hexyl-2,3-dimethylimidazolium, 1-hexyl-3-methylimidazolium, 1-methyl-2-ethylimidazolium, 1-methyl-3-octylimidazolium, 1-methylimidazolium, 1-pentyl-3-methylimidazolium, 1-phenylpropyl-3-methylimidazolium, 1-propyl-2,3-dimethylimidazolium, 1-tetradecyl-3-methylimidazolium, 2,3-dimethylimidazolium, 2-ethyl-3,4-dimethylimidazolium, 3,4-dimethylimidazolium, 1,2-dimethylpyridinium, 1-butyl-2-ethyl-6-methylpyridinium, 1-butyl-2-ethylpyridinium, 1-butyl-2-methylpyridinium, 1-butyl-3,4-dimethylpyridinium, 1-butyl-3,5-dimethylpyridinium, 1-butyl-3-ethylpyridinium, 1-butyl-3-methylpyridinium, 1-butyl-4-methylpyridinium, 1-butylpyridinium, 1-ethylpyridinium, 1-hexyl-3-methylpyridinium, 1-hexyl-4-methylpyridinium, 1-hexylpyridinium, 1-methylpyridinium, 1-octylpyridinium, 2-ethyl-1,6-dimethylpyridinium, 2-ethyl-1-methylpyridinium, 4-methyl-1-octylpyridinium, 1,1-dimethylpyrrolidinium, 1-butyl-1-ethylpyrrolidinium, 1-butyl-1-methylpyrrolidinium, 1-ethyl-1-methylpyrrolidinium, 1-ethyl-3-methylpyrrolidinium, 1-hexyl-1-methylpyrrolidinium, 1-octyl-1-methylpyrrolidinium, guanidinium, hexamethylguanidinium, N,N,N',N'-tetramethyl-N''-ethylguanidinium, N-pentamethyl-N-isopropylguanidinium, N-pentamethyl-N-propylguanidinium, benzyltriphenylphosphonium, tetrabutylphosphonium, trihexyl(tetradecyl)phosphonium, triisobutyl(methyl)phosphonium, butyltrimethylammonium, methyltrioctylammonium, octyltrimethylammonium, tetrabutylammonium, tetraethylammonium, tetramethylammonium, tributylmethylammonium.

3. The process according to claim 1, wherein the cation is selected from the group consisting of 1,2,3-trimethylimidazolium, 1,2-dimethylimidazolium, 1-butyl-2-methylimidazolium, 1-butyl-4-methylimidazolium, 1,3-diethylimidazolium, 1-benzyl-3-methylimidazolium, 1-butyl-2,3-dimethylimidazolium, 1-butyl-2-methylimidazolium, 1-butyl-3-ethylimidazolium, 1-butyl-3-methylimidazolium, 1-butylimidazolium, 1-ethyl-2,3-dimethylimidazolium, 1-ethyl-3-methylimidazolium, 1-hexyl-3-methylimidazolium, 1-methyl-2-ethylimidazolium, 1-methyl-3-octylimidazolium, 1-methylimidazolium, 1-decyl-3-methylimidazolium, 1-dodecyl-3-methylimidazolium, 1-butyl-4-methylpyridinium, 1-butylpyridinium, 1-ethylpyridinium, 1-hexylpyridinium, 1-butyl-1-ethylpyrrolidinium, 1-butyl-1-methylpyrrolidinium, 1-ethyl-1-methylpyrrolidinium, 1-hexyl-1-methylpyrrolidinium, guanidinium, N,N,N',N'-tetramethyl-N''-ethylguanidinium, benzyltriphenylphosphonium, tetrabutylphosphonium, butyltrimethylammonium, methyltrioctylammonium, tetrabutylammonium, tributylmethylammonium.

4. The process according to claim 1, wherein the cation is selected from the group consisting of 1,2,3-trimethylimidazolium, 1,2-dimethylimidazolium, 1-butyl-2,3-dimethylimidazolium, 1-butyl-3-methylimidazolium, 1-butylimidazolium, 1-methylimidazolium, 1-ethyl-2,3-dimethylimidazolium, 1-ethyl-3-methylimidazolium, 1-butyl-4-methylpyridinium, 1-butylpyridinium, methyltrioctylammonium, octyltrimethylammonium.

5. The process according to claim 1, wherein the anion is selected from the group consisting of halides and halogen-containing compounds, sulfates, sulfites and sulfonates, phosphates, phosphites, phosphonites and phosphinites, carboxylic acids, borates, boronates, carbonates and carbonic esters, silicates and silicic esters, alkylsilane and arylsilane salts, carboximides, bis(sulfonyl)imides and sulfonylimides, alkoxides and aryloxides and complex metal ions.

6. The process according to claim 1, wherein the anion is selected from the group consisting of acetate, bis(2,4,4-trimethylpentyl)phosphinate, bis(malonato)borate, bis(oxalato)borate, bis(pentafluoroethyl)phosphinate, bis(phthalato)borate, bis(salicylato)borate, bis(trifluoromethanesulfonyl)imidate, bis(trifluoromethanesulfonyl)methane, bis(trifluoromethyl)imidate, borate, bromide, bromoaluminates, carbonate, chloride, chloroaluminates, decylbenzenesulfonate, dichlorocuprate, dicyanamide, didecylbenzenesulfonate, didodecylbenzenesulfonate, diethylphosphate, dihydrogenphosphate, dodecylbenzenesulfonate, ethylsulfate, ethylsulfonate, fluoride, hexafluorophosphate, hydrogencarbonate, hydrogenphosphate, hydrogensulfate, hydrogensulfite, iodide, methylsulfate, methylsulfonate, nitrate, nitrite, phosphate, sulfate, sulfite, tetracyanoborate, tetrafluoroborate, tetrakis(hydrogensulfato)borate, tetrakis(methylsulfonato)borate, thiocyanate, tosylate, trichlorozincate, trifluoroacetate, trifluoromethylsulfonate, tris(heptafluoropropyl)trifluorophosphate, tris(nonafluorobutyl)trifluorophosphate, tris(pentafluoroethyl)trifluorophosphate, tris(pentafluoroethylsulfonyl)trifluorophosphate.

7. The process according to claim 1, wherein the anion is selected from the group consisting of bis(2,4,4-trimethylpentyl)phosphinate, bis(malonato)borate, bis(oxalato)borate, bis(phthalato)borate, bis(trifluoromethanesulfonyl)imidate, borate, chloride, chloroaluminates, decylbenzenesulfonate, didecylbenzenesulfonate, didodecylbenzenesulfonate, dihydrogenphosphate, dodecylbenzenesulfonate, ethylsulfate, ethylsulfonate, hydrogensulfate, methylsulfate, methylsulfonate, phosphate, sulfate, tetrakis(methylsulfonato)borate, tosylate, trichlorozincate.

8. The process according to claim 1, wherein the anion is selected from the group consisting of chloride, chloroaluminates, ethylsulfate, methylsulfate, methylsulfonate, sulfate, tosylate.

9. The process according to claim 1, wherein the ionic liquid is selected from the group consisting of 1,2,3-trimethylimidazolium chloride, 1,2-dimethylimidazolium chloride, 1-butyl-2,3-dimethylimidazolium chloride, 1-butyl-3-methylimidazolium chloride, 1-butylimidazolium chloride, 1-methylimidazolium chloride, 1-ethyl-2,3-dimethylimidazolium chloride, 1-ethyl-3-methylimidazolium chloride, 1-butyl-4-methylpyridinium chloride, 1-butylpyridinium chloride, methyltrioctylammonium chloride, octyltrimethylammonium chloride, 1,2,3-trimethylimidazolium tetrachloroaluminate, 1,2-dimethylimidazolium tetrachloroaluminate, 1-butyl-2,3-dimethylimidazolium tetrachloroaluminate, 1-butyl-3-methylimidazolium tetrachloroaluminate, 1-ethyl-2,3-dimethylimidazolium tetrachloroaluminate, 1-ethyl-3-methylimidazolium tetrachloroaluminate, 1-butyl-4-methylpyridinium tetrachloroaluminate, 1-butylpyridinium tetrachloroaluminate, methyltrioctylammonium tetrachloroaluminate, octyltrimethylammonium tetrachloroaluminate, 1,2,3-trimethylimidazolium ethylsulfate, 1,2-dimethylimidazolium ethylsulfate, 1-butyl-2,3-dimethylimidazolium ethylsulfate, 1-butyl-3-methylimidazolium ethylsulfate, 1-ethyl-2,3-dimethylimidazolium ethylsulfate, 1-ethyl-3-methylimidazolium ethylsulfate, 1-butyl-4-methylpyridinium ethylsulfate, 1-butylpyridinium ethylsulfate, methyltrioctylammonium ethylsulfate, octyltrimethylammonium ethylsulfate, 1,2,3-trimethylimidazolium methylsulfate, 1,2-dimethylimidazolium methylsulfate, 1-butyl-2,3-dimethylimidazolium methylsulfate, 1-butyl-3-methylimidazolium methylsulfate, 1-ethyl-2,3-dimethylimidazolium methylsulfate, 1-ethyl-3-methylimidazolium methylsulfate, 1-butyl-4-methylpyridinium methylsulfate, 1-butylpyridinium methylsulfate, methyltrioctylammonium methylsulfate, octyltrimethylammonium methylsulfate, 1,2,3-trimethylimidazolium methylsulfonate, 1,2-dimethylimidazolium methylsulfonate, 1-butyl-2,3-dimethylimidazolium methylsulfonate, 1-butyl-3-methylimidazolium methylsulfonate, 1-ethyl-2,3-dimethylimidazolium methylsulfonate, 1-ethyl-3-methylimidazolium methylsulfonate, 1-butyl-4-methylpyridinium methylsulfonate, 1-butylpyridinium methylsulfonate, methyltrioctylammonium methylsulfonate, octyltrimethylammonium methylsulfonate, 1,2,3-trimethylimidazolium sulfate, 1,2-dimethylimidazolium sulfate, 1-butyl-2,3-dimethylimidazolium sulfate, 1-butyl-3-methylimidazolium sulfate, 1-ethyl-2,3-dimethylimidazolium sulfate, 1-ethyl-3-methylimidazolium sulfate, 1-butyl-4-methylpyridinium sulfate, 1-butylpyridinium sulfate, methyltrioctylammonium sulfate, octyltrimethylammonium sulfate, 1,2,3-trimethylimidazolium tosylate, 1,2-dimethylimidazolium tosylate, 1-butyl-2,3-dimethylimidazolium tosylate, 1-butyl-3-methylimidazolium tosylate, 1-ethyl-2,3-dimethylimidazolium tosylate, 1-ethyl-3-methylimidazolium tosylate, 1-butyl-4-methylpyridinium tosylate, 1-butylpyridinium tosylate, methyltrioctylammonium tosylate, octyltrimethylammonium tosylate.

10. The process according to claim 1, wherein the ionic liquid is selected from the group consisting of 1-butyl-2,3-dimethylimidazolium chloride, 1-butyl-3-methylimidazolium chloride, 1-butylimidazolium chloride, 1-methylimidazolium chloride, 1-ethyl-2,3-dimethylimidazolium chloride, 1-ethyl-3-methylimidazolium chloride, 1-butyl-4-methylpyridinium chloride, 1-methylpyridinium chloride, methyltrioctylammonium chloride, 1-butyl-2,3-dimethylimidazolium tetrachloroaluminate, 1-butyl-3-methylimidazolium tetrachloroaluminate, 1-ethyl-2,3-dimethylimidazolium tetrachloroaluminate, 1-ethyl-3-methylimidazolium tetrachloroaluminate, 1-butyl-4-methylpyridinium tetrachloroaluminate, 1-methylpyridinium tetrachloroaluminate, methyltrioctylammonium tetrachloroaluminate, 1-butyl-2,3-dimethylimidazolium ethylsulfate, 1-butyl-3-methylimidazolium ethylsulfate, 1-ethyl-2,3-dimethylimidazolium ethylsulfate, 1-ethyl-3-methylimidazolium ethylsulfate, 1-butyl-4-methylpyridinium ethylsulfate, 1-methylpyridinium ethylsulfate, methyltrioctylammonium ethylsulfate, 1-butyl-2,3-dimethylimidazolium methylsulfate, 1-butyl-3-methylimidazolium methylsulfate, 1-ethyl-2,3-dimethylimidazolium methylsulfate, 1-ethyl-3-methylimidazolium methylsulfate, 1-butyl-4-methylpyridinium methylsulfate, 1-methylpyridinium methylsulfate, methyltrioctylammonium methylsulfate, 1-butyl-2,3-dimethylimidazolium methylsulfonate, 1-butyl-3-methylimidazolium methylsulfonate, 1-ethyl-2,3-dimethylimidazolium methylsulfonate, 1-ethyl-3-methylimidazolium methylsulfonate, 1-butyl-4-methylpyridinium methylsulfonate, 1-methylpyridinium methylsulfonate, methyltrioctylammonium methylsulfonate.

11. The process according to claim 1, wherein the ionic liquid is selected from the group consisting of 1-butyl-2,3-dimethylimidazolium chloride, 1-butyl-3-methylimidazolium chloride, 1-butylimidazolium chloride, 1-methylimidazolium chloride, 1-ethyl-3-methylimidazolium chloride, 1-butyl-4-methylpyridinium chloride, methyltrioctylammonium chloride, 1-butyl-2,3-dimethylimidazolium tetrachloroaluminate, 1-butyl-3-methylimidazolium tetrachloroaluminate, 1-ethyl-3-methylimidazolium tetrachloroaluminate, 1-butyl-4-methylpyridinium tetrachloroaluminate, methyltrioctylammonium tetrachloroaluminate, 1-butyl-2,3-dimethylimidazolium methylsulfate, 1-butyl-3-methylimidazolium methylsulfate, 1-ethyl-3-methylimidazolium methylsulfate, 1-butyl-4-methylpyridinium methylsulfate, methyltrioctylammonium methylsulfate, 1-butyl-2,3-dimethylimidazolium methylsulfonate, 1-butyl-3-methylimidazolium methylsulfonate, 1-ethyl-3-methylimidazolium methylsulfonate, 1-butyl-4-methylpyridinium methylsulfonate, methyltrioctylammonium methylsulfonate.

12. The process according to claim 1, wherein the ionic liquid is selected from the group consisting of 1-butyl-3-methylimidazolium chloride, 1-ethyl-3-methylimidazolium chloride, 1-butyl-4-methylpyridinium chloride, 1-butylimidazolium chloride, 1-methylimidazolium chloride.

13. The process according to claim 1, wherein the amine is present as a solution of amine hydrochloride in the ionic liquid.

14. The process according to claim 1, wherein the ionic liquid is used in admixture with hydrogen chloride.

15. The process according to claim 14, wherein the ratio of hydrogen chloride to ionic liquid is from > 0 to 400 mol%.

16. The process according to claim 14, wherein the ratio of hydrogen chloride to ionic liquid is from 5 to 300 mol%.

17. The process according to claim 14, wherein the ratio of hydrogen chloride to ionic liquid is from 10 to 150 mol%.

## Revendications

1. Procédé pour la préparation de polyisocyanates par mise en réaction d'amines primaires avec du phosgène en présence d'un solvant, **caractérisé en ce qu'**on utilise comme solvant des liquides ioniques qui comportent au moins un cation et au moins un anion, et le cation du liquide ionique est organique, le cation du liquide ionique étant choisi dans le groupe contenant des cations ammonium quaternaire, des cations phosphonium, des cations imidazolium, des cations H-pyrazolium, des ions pyridazinium, des ions pyrimidinium, des ions pyrazinium, des cations pyrrolidinium, des cations guanidinium, des cations à cinq à au moins six chaînons, qui contiennent au moins un atome de phosphore ou de soufre, et le cation 1,8-diazabicyclo[5.4.0]undéc-7-énium ainsi que le cation 1,8-diazabicyclo[4.3.0]non-5-énium ainsi que des oligo- et polymères qui contiennent ces cations.

2. Procédé selon la revendication 1, **caractérisé en ce que** le cation est choisi dans le groupe contenant les cations 1,2,3-triméthylimidazolium, 1,3,4,5-tétraméthylimidazolium, 1,3,4-diméthylimidazolium, 1,3,4-triméthylimidazolium, 1,3-dibutyl-2-méthylimidazolium, 1,3-dibutylimidazolium, 1,2-diméthylimidazolium, 1,3-diméthylimidazolium, 1-benzyl-3-méthylimidazolium, 1-butyl-2,3-diméthylimidazolium, 1-butyl-2-éthyl-5-méthylimidazolium, 1-butyl-2-éthylimidazolium, 1-butyl-2-méthylimidazolium, 1-butyl-3,4,5-triméthylimidazolium, 1-butyl-3,4-diméthylimidazolium, 1-butyl-3-éthylimidazolium, 1-butyl-3-méthylimidazolium, 1-butyl-4-méthylimidazolium, 1-butylimidazolium, 1-décyl-3-méthylimidazolium, 1-dodécyl-3-méthylimidazolium, 1-éthyl-2,3-diméthylimidazolium, 1-éthyl-3-méthylimidazolium, 1-hexadécyl-2,3-diméthylimidazolium, 1-hexadécyl-3-méthylimidazolium, 1-hexyl-2,3-diméthylimidazolium, 1-hexyl-3-méthylimidazolium, 1-méthyl-2-éthylimidazolium, 1-méthyl-3-octylimidazolium, 1-méthylimidazolium, 1-pentyl-3-méthylimidazolium, 1-phénylpropyl-3-méthylimidazolium, 1-propyl-2,3-diméthylimidazolium, 1-tétradécyl-3-méthylimidazolium, 2,3-diméthylimidazolium, 2-éthyl-3,4-diméthylimidazolium, 3,4-diméthylimidazolium, 1,2-diméthylpyridinium, 1-butyl-2-éthyl-6-méthylpyridinium, 1-butyl-2-éthylpyridinium, 1-butyl-2-méthylpyridinium, 1-butyl-3,4-diméthylpyridinium, 1-butyl-3,5-diméthylpyridinium, 1-butyl-3-éthylpyridinium, 1-butyl-3-méthylpyridinium, 1-butyl-4-méthylpyridinium, 1-butylpyridinium, 1-éthylpyridinium, 1-hexyl-3-méthylpyridinium, 1-hexyl-4-méthylpyridinium, 1-hexylpyridinium, 1-méthylpyridinium, 1-octylpyridinium, 2-éthyl-1,6-diméthylpyridinium, 2-éthyl-1-méthylpyridinium, 4-méthyl-1-octylpyridinium, 1,1-diméthylpyrrolidinium, 1-butyl-1-éthylpyrrolidinium, 1-butyl-1-méthylpyrrolidinium, 1-éthyl-1-méthylpyrrolidinium, 1-éthyl-3-méthylpyrrolidinium, 1-hexyl-1-méthylpyrrolidinium, 1-octyl-1-méthylpyrrolidinium, guanidinium, hexaméthylguanidinium, N,N,N',N'-tétraméthyl-N''-éthylguanidinium, N-pentaméthyl-N-isopropylguanidinium, N-pentaméthyl-N-propylguanidinium, benzyltriphénylphosphonium, tétrabutylphosphonium, trihexyl(tétradécyl)phosphonium, tri-isobutyl(méthyl)phosphonium, butyltriméthylammonium, méthyltrioctylammonium, octyltriméthylammonium, tétrabutylammonium, tétraéthylammonium, tétraméthylammonium, tributylméthylammonium.

3. Procédé selon la revendication 1, **caractérisé en ce que** le cation est choisi dans le groupe contenant les cations 1,2,3-triméthylimidazolium, 1,2-diméthylimidazolium, 1-butyl-2-méthylimidazolium, 1-butyl-4-méthylimidazolium, 1,3-diéthylimidazolium, 1-benzyl-3-méthylimidazolium, 1-butyl-2,3-diméthylimidazolium, 1-butyl-2-méthylimidazolium, 1-butyl-3-éthyl-imidazolium, 1-butyl-3-méthylimidazolium, 1-butylimidazolium, 1-éthyl-2,3-diméthylimidazolium, 1-éthyl-3-méthylimidazolium, 1-hexyl-3-méthylimidazolium, 1-méthyl-2-éthylimidazolium, 1-méthyl-3-octyl-imidazolium, 1-méthylimidazolium, 1-décyl-3-méthylimidazolium, 1-dodécyl-3-méthylimidazolium, 1-butyl-4-méthylpyridinium, 1-butylpyridinium, 1-éthylpyridinium, 1-hexylpyridinium, 1-butyl-1-éthylpyrrolidinium, 1-butyl-1-méthylpyrrolidinium, 1-éthyl-1-méthyl-pyrrolidinium, 1-hexyl-1-méthylpyrrolidinium, guanidinium, N,N,N',N'-tétraméthyl-N''-éthyl-guanidinium, benzyltriphénylphosphonium, tétrabutylphosphonium, butyltriméthylammonium, méthyltrioctylammonium, tétrabutylammonium, tributylméthylammonium.

4. Procédé selon la revendication 1, **caractérisé en ce que** le cation est choisi dans le groupe contenant les cations 1,2,3-triméthylimidazolium, 1,2-diméthylimidazolium, 1-butyl-2,3-diméthylimidazolium, 1-butyl-3-méthylimidazolium, 1-butylimidazolium, 1-méthylimidazolium, 1-éthyl-2,3-diméthylimidazolium, 1-éthyl-3-méthylimidazolium, 1-butyl-4-méthylpyridinium, 1-butylpyridinium, méthyltrioctylammonium, octyltriméthylammonium.

5. Procédé selon la revendication 1, **caractérisé en ce que** l'anion est choisi dans le groupe contenant les halogénures ou composés halogénés, sulfates, sulfites et sulfonates, phosphates, phosphites, phosphonites et phosphinites, acides carboxyliques, borates, boronates, carbonates et esters d'acide carbonique, silicates et esters d'acide silicique, sels d'alkyl- ou arylsilane, carboximides, bis(sulfonyl)imides et sulfonylimides, alcoolates et aryloxydes ainsi que des ions métalliques complexes.

6. Procédé selon la revendication 1, **caractérisé en ce que** l'anion est choisi dans le groupe contenant les anions acétate, bis(2,4,4-triméthylpentyl)-phosphinate, bis(malonato)borate, bis(oxalato)borate, bis(pentafluoroéthyl)phosphinate, bis(phtalato)borate, bis(salicylato)borate, bis(trifluorométhanesulfonyl)-imidate, bis(trifluorométhanesulfonyl)méthane, bis(trifluorométhyl)imidate, borate, bromure, bromoaluminates, carbonate, chlorure, chloroaluminates, décylbenzènesulfonate, dichlorocuprate, dicyanamide, didécylbenzènesulfonate, didodécylbenzènesulfonate, diéthylphosphate, dihydrogénophosphate, dodécylbenzènesulfonate, éthylsulfate, éthylsulfonate, fluorure, hexafluorophosphate, hydrogénocarbonate, hydrogénophosphate, hydrogénosulfate, hydrogénosulfite, iodure, méthylsulfate, méthylsulfonate, nitrate, nitrite, phosphate, sulfate, sulfite, tétracyanoborate, tétrafluoroborate, tétrakis(hydrogénosulfato)borate, tétrakis(méthylsulfonato)borate, thiocyanate, tosylate, trichlorozincate, trifluoroacétate, trifluorométhylsulfonate, tris(heptafluoropropyl)-trifluorophosphate, tris(nonafluorobutyl)trifluoro-phosphate, tris(pentafluoroéthyl)trifluorophosphate, tris(pentafluoroéthylsulfonyl)trifluorophosphate.

7. Procédé selon la revendication 1, **caractérisé en ce que** l'anion est choisi dans le groupe contenant les anions bis(2,4,4-triméthylpentyl)phosphinate, bis(malonato)borate, bis(oxalato)borate, bis(phtalato)-borate, bis(trifluorométhanesulfonyl)imidate, borate, chlorure, chloroaluminates, décylbenzènesulfonate, didécylbenzènesulfonate, didodécylbenzènesulfonate, dihydrogénophosphate, dodécylbenzènesulfonate, éthylsulfate, éthylsulfonate, hydrogénosulfate, méthylsulfate, méthylsulfonate, phosphate, sulfate, tétrakis(méthylsulfonato)borate, tosylate, trichlorozincate.

8. Procédé selon la revendication 1, **caractérisé en ce que** l'anion est choisi dans le groupe contenant les anions chlorure, chloroaluminate, éthylsulfate, méthylsulfate, méthylsulfonate, sulfate, tosylate.

9. Procédé selon la revendication 1, **caractérisé en ce que** le liquide ionique est choisi dans le groupe contenant le chlorure de 1,2,3-triméthylimidazolium, le chlorure de 1,2-diméthylimidazolium, le chlorure de 1-butyl-2,3-diméthylimidazolium, le chlorure de 1-butyl-3-méthylimidazolium, le chlorure de 1-butylimidazolium, le chlorure de 1-méthylimidazolium, le chlorure de 1-éthyl-2,3-diméthylimidazolium, le chlorure de 1-éthyl-3-méthylimidazolium, le chlorure de 1-butyl-4-méthylpyridinium, le chlorure de 1-butylpyridinium, le chlorure de méthyltrioctylammonium, le chlorure d'octyltriméthylammonium, le tétrachloroaluminate de 1,2,3-triméthylimidazolium, le tétrachloroaluminate de 1,2-diméthylimidazolium, le tétrachloroaluminate de 1-butyl-2,3-diméthylimidazolium, le tétrachloroaluminate de 1-butyl-3-méthylimidazolium, le tétrachloroaluminate de 1-éthyl-2,3-diméthylimidazolium, le tétrachloroaluminate de 1-éthyl-3-méthylimidazolium, le tétrachloroaluminate de 1-butyl-4-méthylpyridinium, le tétrachloroaluminate de 1-butylpyridinium, le tétrachloroaluminate de méthyltrioctylammonium, le tétrachloroaluminate d'octyltriméthylammonium, l'éthylsulfate de 1,2,3-triméthylimidazolium, l'éthylsulfate de 1,2-diméthylimidazolium, l'éthylsulfate de 1-butyl-2,3-diméthylimidazolium, l'éthylsulfate de 1-butyl-3-méthylimidazolium, l'éthylsulfate de 1-éthyl-2,3-diméthylimidazolium, l'éthylsulfate de 1-éthyl-3-méthylimidazolium, l'éthylsulfate de 1-butyl-4-méthylpyridinium, l'éthylsulfate de 1-butylpyridinium, l'éthylsulfate de méthyltrioctylammonium, l'éthylsulfate d'octyltriméthylammonium, le méthylsulfate de 1,2,3-triméthylimidazolium, le méthylsulfate de 1,2-diméthylimidazolium, le méthylsulfate de 1-butyl-2,3-diméthylimidazolium, le méthylsulfate de 1-butyl-3-méthylimidazolium, le méthylsulfate de 1-éthyl-2,3-diméthylimidazolium, le méthylsulfate de 1-éthyl-3-méthylimidazolium, le méthylsulfate de 1-butyl-4-méthylpyridinium, le méthylsulfate de 1-butylpyridinium, le méthylsulfate de méthyltrioctylammonium, le méthylsulfate d'octyltriméthylammonium, le méthylsulfonate de 1,2,3-triméthylimidazolium, le méthylsulfonate de 1,2-diméthylimidazolium, le méthylsulfonate de 1-butyl-2,3-diméthylimidazolium, le méthylsulfonate de 1-butyl-3-méthylimidazolium, le méthylsulfonate de 1-éthyl-2,3-diméthylimidazolium, le méthylsulfonate de 1-éthyl-3-méthylimidazolium, le méthylsulfonate de 1-butyl-4-méthylpyridinium, le méthylsulfonate de 1-butylpyridinium, le méthylsulfonate de méthyltrioctylammonium, le méthylsulfonate d'octyltriméthylammonium, le sulfate de 1,2,3-triméthylimidazolium, le sulfate de 1,2-diméthylimidazolium, le sulfate de 1-butyl-2,3-diméthylimidazolium, le sulfate de 1-butyl-3-méthylimidazolium, le sulfate de 1-éthyl-2,3-diméthylimidazolium, le sulfate de 1-éthyl-3-méthylimidazolium, le sulfate de 1-butyl-4-méthylpyridinium, le sulfate de 1-butylpyridinium, le sulfate de méthyltrioctylammonium, le sulfate d'octyltriméthylammonium, le tosylate de 1,2,3-triméthylimidazolium, le tosylate de 1,2-diméthylimidazolium, le tosylate de 1-butyl-2,3-diméthylimidazolium, le tosylate de 1-butyl-3-méthylimidazolium, le tosylate de 1-éthyl-2,3-diméthylimidazolium, le tosylate de 1-éthyl-3-méthylimidazolium, le tosylate de 1-butyl-4-méthylpyridinium, le tosylate de 1-butylpyridinium, le tosylate de méthyltrioctylammonium, le tosylate d'octyltriméthylammonium.

10. Procédé selon la revendication 1, **caractérisé en ce que** le liquide ionique est choisi dans le groupe contenant le chlorure de 1-butyl-2,3-diméthylimidazolium, le chlorure de 1-butyl-3-méthylimidazolium, le chlorure de 1-butylimidazolium, le chlorure de 1-méthylimidazolium, le chlorure de 1-éthyl-2,3-diméthylimidazolium, le chlorure de 1-éthyl-3-méthylimidazolium, le chlorure de 1-butyl-4-méthylpyridinium, le chlorure de 1-méthylpyridinium, le chlorure de méthyltrioctylammonium, le tétrachloroaluminate de 1-butyl-2,3-diméthylimidazolium, le tétrachloroaluminate de 1-butyl-3-méthylimidazolium, le tétrachloroaluminate de 1-éthyl-2,3-diméthylimidazolium, le tétrachloroaluminate de 1-éthyl-3-méthylimidazolium, le tétrachloroaluminate de 1-butyl-4-méthylpyridinium, le tétrachloroaluminate de 1-méthylpyridinium, le tétrachloroaluminate de méthyltrioctylammonium, l'éthylsulfate de 1-butyl-2,3-diméthylimidazolium, l'éthylsulfate de 1-butyl-3-méthylimidazolium, l'éthylsulfate de 1-éthyl-2,3-diméthylimidazolium, l'éthylsulfate de 1-éthyl-3-méthylimidazolium, l'éthylsulfate de 1-butyl-4-méthylpyridinium, l'éthylsulfate de 1-méthylpyridinium, l'éthylsulfate de méthyltrioctylammonium, le méthylsulfate de 1-butyl-2,3-diméthylimidazolium, le méthylsulfate de 1-butyl-3-méthylimidazolium, le méthylsulfate de 1-éthyl-2,3-diméthylimidazolium, le méthylsulfate de 1-éthyl-3-méthylimidazolium, le méthylsulfate de 1-butyl-4-méthylpyridinium, le méthylsulfate de 1-méthylpyridinium, le méthylsulfate de méthyltrioctylammonium, le méthylsulfonate de 1-butyl-2,3-diméthylimidazolium, le méthylsulfonate de 1-butyl-3-méthylimidazolium, le méthylsulfonate de 1-éthyl-2,3-diméthylimidazolium, le méthylsulfonate de 1-éthyl-3-méthylimidazolium, le méthylsulfonate de 1-butyl-4-méthylpyridinium, le méthylsulfonate de 1-méthylpyridinium, le méthylsulfonate de méthyltrioctylammonium.

11. Procédé selon la revendication 1, **caractérisé en ce que** le liquide ionique est choisi dans le groupe contenant le chlorure de 1-butyl-2,3-diméthylimidazolium, le chlorure de 1-butyl-3-méthylimidazolium, le chlorure de 1-butylimidazolium, le chlorure de 1-méthylimidazolium, le chlorure de 1-éthyl-3-méthylimidazolium, le chlorure de 1-butyl-4-méthylpyridinium, le chlorure de méthyltrioctylammonium, le tétrachloroaluminate de 1-butyl-2,3-diméthylimidazolium, le tétrachloroaluminate de 1-butyl-3-méthylimidazolium, le tétrachloroaluminate de 1-éthyl-3-méthylimidazolium, le tétrachloroaluminate de 1-butyl-4-méthylpyridinium, le tétrachloroaluminate de méthyltrioctylammonium, le méthylsulfate de 1-butyl-2,3-diméthylimidazolium, le méthylsulfate de 1-butyl-3-méthylimidazolium, le méthylsulfate de 1-éthyl-3-méthylimidazolium, le méthylsulfate de 1-butyl-4-méthylpyridinium, le méthylsulfate de méthyltrioctylammonium, le méthylsulfonate de 1-butyl-2,3-diméthylimidazolium, le méthylsulfonate de 1-butyl-3-méthylimidazolium, le méthylsulfonate de 1-éthyl-3-méthylimidazolium, le méthylsulfonate de 1-butyl-4-méthylpyridinium, le méthylsulfonate de méthyltrioctylammonium.

12. Procédé selon la revendication 1, **caractérisé en ce que** le liquide ionique est choisi dans le groupe contenant le chlorure de 1-butyl-3-méthylimidazolium, le chlorure de 1-éthyl-3-méthylimidazolium, le chlorure de 1-butyl-4-méthylpyridinium, le chlorure de 1-butylimidazolium, le chlorure de 1-méthylimidazolium.

13. Procédé selon la revendication 1, **caractérisé en ce que** l'amine est présente sous forme de chlorhydrate d'amine en solution dans le liquide ionique.

14. Procédé selon la revendication 1, **caractérisé en ce que** le liquide ionique est utilisé en mélange avec de l'acide chlorhydrique.

15. Procédé selon la revendication 14, **caractérisé en ce que** le rapport de l'acide chlorhydrique au liquide ionique vaut de plus de 0 à 400 % en moles.

16. Procédé selon la revendication 14, **caractérisé en ce que** le rapport de l'acide chlorhydrique au liquide ionique vaut de 5 à 300 % en moles.

17. Procédé selon la revendication 14, **caractérisé en ce que** le rapport de l'acide chlorhydrique au liquide ionique vaut de 10 à 150 % en moles.
